(19) Europäisches Patentamt / European Patent Office / Office européen des brevets

(11) EP 1 562 650 B1

(12) **EUROPÄISCHE PATENTSCHRIFT**

(45) Veröffentlichungstag und Bekanntmachung des Hinweises auf die Patenterteilung:
**14.02.2007 Patentblatt 2007/07**

(21) Anmeldenummer: **03753507.7**

(22) Anmeldetag: **06.10.2003**

(51) Int Cl.:
***A61L 15/60*** *(2006.01)* ***C08J 9/28*** *(2006.01)*
***C08J 9/00*** *(2006.01)*

(86) Internationale Anmeldenummer:
**PCT/EP2003/011013**

(87) Internationale Veröffentlichungsnummer:
**WO 2004/035668 (29.04.2004 Gazette 2004/18)**

(54) **SUPERABSORBIERENDER SCHAUM, VERFAHREN ZU SEINER HERSTELLUNG UND SEINE VERWENDUNG**

SUPERABSORBING FOAM, METHOD FOR THE PRODUCTION THEREOF, AND USE THEREOF

MOUSSE SUPERABSORBANTE, PROCEDE DE FABRICATION DE LADITE MOUSSE ET SON UTILISATION

(84) Benannte Vertragsstaaten:
**AT BE BG CH CY CZ DE DK EE ES FI FR GB GR HU IE IT LI LU MC NL PT RO SE SI SK TR**

(30) Priorität: **10.10.2002 DE 10247241**

(43) Veröffentlichungstag der Anmeldung:
**17.08.2005 Patentblatt 2005/33**

(73) Patentinhaber: **BASF Aktiengesellschaft**
**67056 Ludwigshafen (DE)**

(72) Erfinder:
- **CHAMP, Samantha**
  **67063 Ludwigshafen (DE)**
- **WANIOR, Mariola**
  **63526 Erlensee (DE)**
- **REIFSCHNEIDER, Andreas**
  **68199 Mannheim (DE)**

(56) Entgegenhaltungen:
**WO-A-97/31971** **US-A1- 2002 068 494**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 562 650 B1

## Beschreibung

Die Erfindung betrifft einen superabsorbierenden Schaum, der erhältlich ist durch Schäumen einer polymerisierbaren wäßrigen Mischung und Polymerisieren der geschäumten Mischung, ein Verfahren zur Herstellung eines superabsorbierenden Schaums und die Verwendung des Schaums in Hygieneartikeln zur Absorption von Körperflüssigkeiten.

Wasserabsorbierende, überwiegend offenzellige Schäume auf Basis von vernetzten Säuregruppen enthaltenden Monomeren sind bekannt, vgl. EP-B-0 858 478, WO-A-99/44648 und WO-A-00/52087. Sie werden beispielsweise durch Schäumen einer polymerisierbaren wäßrigen Mischung, die zu mindestens 50 Mol-% neutralisierte, Säuregruppen enthaltende monoethylenisch ungesättigte Monomere, Vernetzer und mindestens ein Tensid enthalten, und anschließendes Polymerisieren der geschäumten Mischung hergestellt. Das Schäumen der polymerisierbaren Mischung kann z.B. durch Dispergieren von feinen Blasen eines gegenüber Radikalen inerten Gases oder durch Lösen eines solchen Gases unter erhöhtem Druck in der polymerisierbaren Mischung und Entspannen der Mischung erfolgen. Der Wassergehalt der Schaumstoffe wird auf beispielsweise 1 bis 60 Gew.-% eingestellt. Die Schäume können gegebenenfalls einer Oberflächennachvernetzung unterworfen werden, indem man einen Vernetzer auf das geschäumte Material sprüht oder den Schaum darin eintaucht und den mit Vernetzer beladenen Schaum auf eine höhere Temperatur erhitzt. Die Schäume werden z.B. in Hygieneartikeln zur Akquisition, Distribution und Speicherung von Körperflüssigkeiten verwendet.

Aus der WO-A-97/31600 ist ein Absorberelement für den Einsatz in Hygiene- oder Sanitärartikeln bekannt, wobei mehrere Elemente aus einem superabsorbierenden Schaum rasterförmig auf einem Träger in solchen Abständen angeordnet sind, daß sich die Elemente in gequollenem Zustand an ihren Umfängen berühren. Man kann beispielsweise einen Monomerschaum in dem gewünschten Rastermuster auf den Träger aufbringen und anschließend darauf polymerisieren oder separat hergestellte Schaumelemente auf dem Träger chemisch oder physikalisch in dem gewünschten Rastermuster fixieren. Die Naßfestigkeit der oben beschriebenen superabsorbierenden Schäume ist jedoch noch verbesserungsbedürftig.

Außerdem sind superabsorbierende Fasern bekannt, die beispielsweise dadurch erhältlich sind, daß man die Carboxylgruppen eines hydrolysierten Copolymerisats aus Isobuten und Maleinsäureanhydrid zu 20 bis 80% mit Natronlauge neutralisiert, eine bifunktionelle Verbindung zusetzt, die mit den nicht neutralisierten Carboxylgruppen des Copolymerisats reagieren kann, z.B. Propylenglykol oder Ethanolamin, dann das Wasser aus der Lösung weitgehend entfernt, so daß der Feststoffgehalt der Lösung 45% beträgt. Diese Lösung wird anschließend zu Fasern versponnen. Die Fasern werden danach auf eine höhere Temperatur erhitzt, z.B. auf 210°C, wobei die Copolymerisate vernetzt werden. Die vernetzten Copolymerisate haben superabsorbierende Eigenschaften. Sie werden beispielsweise in Babywindeln, Tampons, Monatsbinden, chirurgischen Schwämmen und Verbänden zur Absorption von Körperflüssigkeiten verwendet. Solche superabsorbierenden Fasern sind bekannt, vgl. beispielsweise EP-B-0 264 208, EP-B-0 272 072, EP-B-0 436 514 und US-A-4,813,945.

Aus der älteren, nicht vorveröffentlichten DE-Anmeldung 102 04 980.7 sind Schaumstoffe aus wasserabsorbierenden basischen Polymeren bekannt, die durch Schäumen einer wäßrigen Mischung, die mindestens ein basisches Polymer wie Polyvinylamin und mindestens einen Vernetzer wie Glycidylether, enthält, und anschließendes Vernetzen der geschäumten Mischung erhältlich sind. Die Naßfestigkeit der so erhältlichen wasserabsorbierenden Schäume ist ebenfalls noch verbesserungsbedürftig.

Der Erfindung liegt die Aufgabe zugrunde, die Naßfestigkeit von wasserabsorbierenden Schäumen zu verbessern.

Die Aufgabe wird erfindungsgemäß gelöst mit superabsorbierenden Schäumen, die superabsorbierende synthetische Fasern und/oder natürliche Fasern aus der Gruppe der Apfelfasern, Orangenfasern, Tomatenfasern, Weizenfasern und/oder Haferfasern enthalten. Solche Schäume sind erhältlich durch Schäumen einer polymerisierbaren wäßrigen Mischung, die zu mindestens 50 Mol-% neutralisierte, Säuregruppen enthaltende monoethylenisch ungesättigte Monomere oder mindestens ein basisches Polymer, Vernetzer, superabsorbierende Fasern und mindestens ein Tensid enthält, und anschließendes Polymerisieren und/oder Vernetzen der geschäumten Mischung.

Gegenstand der Erfindung ist außerdem ein Verfahren zur Herstellung von superabsorbierenden Schäumen mit verbesserter Naßfestigkeit, wobei man eine vernetzbare wäßrige Mischung schäumt, die zu mindestens 50 Mol-% neutralisierte, Säuregruppen enthaltende monoethylenisch ungesättigte Monomere oder mindestens ein basisches Polymer, Vernetzer, superabsorbierende synthetische Fasern und/oder natürliche Fasern aus der Gruppe der Apfelfasern, Oragenfasern, Tomatenfasern, Weizenfasern und/oder Haferfasern und mindestens ein Tensid enthält, und anschließend die in der geschäumten Mischung enthaltenen Monomeren polymerisiert oder die basischen Polymeren unter Bildung eines schaumförmigen Hydrogels vernetzt.

Schaumstoffe auf Basis von vernetzten, Säuregruppen enthaltenden Polymerisaten sind aus den zum Stand der Technik genannten Literaturstellen EP-B-0 858 478, Seite 2, Zeile 55 bis Seite 18, Zeile 22, WO-A-99/44648 und WO-A-00/52087, Seite 5, Zeile 23 bis Seite 41, Zeile 18 bekannt. Nach den bekannten Verfahren schäumt man zunächst

eine wäßrige Mischung, die beispielsweise

a) 10 bis 80 Gew.-% Säuregruppen enthaltende monoethylenisch ungesättigte Monomerem die zu mindestens 50 Mol-% neutralisiert sind,
b) gegebenenfalls bis zu 50 Gew.-% andere monoethylenisch ungesättigte Monomere,
c) 0,001 bis 5 Gew.-% Vernetzer,
d) Initiatoren,
e) 0,1 bis 20 Gew.-% mindestens eines Tensids,
f) gegebenenfalls einen Lösevermittler und
g) gegebenenfalls Verdicker, Schaumstabilisatoren, Polymerisationsregler, Füllstoffe und/oder Zellkeimbildner

enthält. Man kann jedoch auch eine wäßrige Mischung schäumen, die anstelle der Monomeren (a) und (b) eine basisches Polymer enthält, dessen basische Gruppen gegebenenfalls teilweise neutralisiert sind. Das Schäumen der wäßrigen Mischungen kann beispielsweise dadurch erfolgen, daß man feine Blasen eines gegenüber Radikalen inerten Gases in der Mischung dispergiert oder ein solches Gas unter einem Druck von 2 bis 400 bar in der vernetzbaren Mischung löst und sie anschließend auf Atmosphärendruck entspannt. Man erhält einen fließfähigen Schaum, der in Formen eingefüllt oder auf einem Band ausgehärtet werden kann. Das Aushärten erfolgt im Fall des Einsatzes von Säuregruppen enthaltenden Monomeren, gegebenenfalls anderen monoethylenisch ungesättigten Monomeren und Vernetzern durch Polymerisation und im Fall des Einsatzes von basischen Polymeren unter Vernetzung.

Basische Polymere

Als basische Polymere kommen beispielsweise Vinylamineinheiten enthaltende Polymere, Vinylguanidineinheiten enthaltende Polymere, Dialkylaminoalkyl(meth)acrylamideinheiten enthaltende Polymere, Polyethylenimine, mit Ethylenimin gepfropfte Polyamidoamine und Polydiallyldimethylammoniumchloride in Betracht.
Vinylamineinheiten enthaltende Polymere sind bekannt, vgl. US-A-4,421,602, US-A-5,334,287, EP-A-0 216 387, US-A-5,981,689, WO-A-00/63295 und US-A-6,121,409. Sie werden durch Hydrolyse von offenkettigen N-Vinylcarbonsäureamideinheiten enthaltenden Polymeren hergestellt. Diese Polymeren sind z.B. erhältlich durch Polymerisieren von N-Vinylformamid, N-Vinyl-N-methylformamid, N-Vinylacetamid, N-Vinyl-N-methylacetamid, N-Vinyl-N-ethylacetamid und N-Vinylpropionamid. Die genannten Monomeren können entweder allein oder zusammen mit anderen Monomeren polymerisiert werden.
Als monoethylenisch ungesättigte Monomere, die mit den N-Vinylcarbonsäureamiden copolymerisiert werden, kommen alle damit copolymerisierbaren Verbindungen in Betracht. Beispiele hierfür sind Vinylester von gesättigten Carbonsäuren von 1 bis 6 Kohlenstoffatomen wie Vinylformiat, Vinylacetat, Vinylpropionat und Vinylbutyrat und Vinylether wie $C_1$- bis $C_6$-Alkylvinylether, z.B. Methyl- oder Ethylvinylether. Weitere geeignete Comonomere sind Ester, Amide und Nitrile von ethylenisch ungesättigten $C_3$- bis $C_6$-Carbonsäuren, beispielsweise Methylacrylat, Methylmethacrylat, Ethylacrylat und Ethylmethacrylat, Acrylamid und Methacrylamid sowie Acrylnitril und Methacrylnitril.
Weitere geeignete Carbonsäureester leiten sich von Glykolen oder bzw. Polyalkylenglykolen ab, wobei jeweils nur eine OH-Gruppe verestert ist, z.B. Hydroxyethylacrylat, Hydroxyethylmethacrylat, Hydroxypropylacrylat, Hydroxybutylacrylat, Hydroxypropylmethacrylat, Hydroxybutylmethacrylat sowie Acrylsäuremonoester von Polyalkylenglykolen einer Molmasse von 500 bis 10000. Weitere geeignete Comonomere sind Ester von ethylenisch ungesättigten Carbonsäuren mit Aminoalkoholen wie beispielsweise Dimethylaminoethylacrylat, Dimethylaminoethylmethacrylat, Diethylaminoethylacrylat, Diethylaminoethylmethacrylat, Dimethylaminopropylacrylat, Dimethylaminopropylmethacrylat, Diethylaminopropylacrylat, Dimethylaminobutylacrylat und Diethylaminobutylacrylat. Die basischen Acrylate können in Form der freien Basen, der Salze mit Mineralsäuren wie Salzsäure, Schwefelsäure oder Salpetersäure, der Salze mit organischen Säuren wie Ameisensäure, Essigsäure, Propionsäure oder der Sulfonsäuren oder in quaternierter Form eingesetzt werden. Geeignete Quaternierungsmittel sind beispielsweise Dimethylsulfat, Diethylsulfat, Methylchlorid, Ethylchlorid oder Benzylchlorid.
Weitere geeignete Comonomere sind Amide ethylenisch ungesättigter Carbonsäuren wie Acrylamid, Methacrylamid sowie N-Alkylmono- und Diamide von monoethylenisch ungesättigten Carbonsäuren mit Alkylresten von 1 bis 6 C-Atomen, z.B. N-Methylacrylamid, N,N-Dimethylacrylamid, N-Methylmethacrylamid, N-Ethylacrylamid, N-Propylacrylamid und tert. Butylacrylamid sowie basische (Meth)acrylamide, wie z.B. Dimethylaminoethylacrylamid, Dimethylaminoethylmethacrylamid, Diethylaminoethylacrylamid, Diethylaminoethylmethacrylamid, Dimethylaminopropylacrylamid, Diethylaminopropylacrylamid, Dimethylaminopropylmethacrylamid und Diethylaminopropylmethacrylamid.
Weiterhin sind als Comonomere geeignet N-Vinylpyrrolidon, N-Vinylcaprolactam, Acrylnitril, Methacrylnitril, N-Vinylimidazol sowie substituierte N-Vinylimidazole wie z.B. N-Vinyl-2-methylimidazol, N-Vinyl-4-methylimidazol, N-Vinyl-5-methylimidazol, N-Vinyl-2-ethylimidazol und N-Vinylimidazoline wie N-Vinylimidazolin, N-Vinyl-2-methylimidazolin und N-Vinyl-2-ethylimidazolin. N-Vinylimidazole und N-Vinylimidazoline werden außer in Form der freien Basen auch in mit

Mineralsäuren oder organischen Säuren neutralisierter oder in quaternisierter Form eingesetzt, wobei die Quaternisierung vorzugsweise mit Dimethylsulfat, Diethylsulfat, Methylchlorid oder Benzylchlorid vorgenommen wird. In Frage kommen auch Diallyldialkylammoniumhalogenide wie z.B. Diallyldimethylammoniumchlorid.

Die Copolymerisate enthalten beispielsweise

- 95 bis 5 mol-%, vorzugsweise 90 bis 10 mol-% mindestens eines N-Vinylcarbonsäureamids und
- 5 bis 95 mol-%, vorzugsweise 10 bis 90 mol-% andere, damit copolymerisierbare monoethylenisch ungesättigte Monomere

in einpolymerisierter Form. Die Comonomeren sind vorzugsweise frei von Säuregruppen.

Um Vinylamineinheiten enthaltende Polymerisate herzustellen, geht man vorzugsweise von Homopolymerisaten des N-Vinylformamids oder von Copolymerisaten aus, die durch Copolymerisieren von

- N-Vinylformamid mit
- Vinylformiat, Vinylacetat, Vinylpropionat, Acrylnitril, N-Vinylcaprolactam, N-Vinylharnstoff, N-Vinylpyrrolidon oder $C_1$- bis $C_6$-Alkylvinylethern

und anschließende Hydrolyse der Homo- oder der Copolymerisate unter Bildung von Vinylamineinheiten aus den einpolymerisierten N-Vinylformamideinheiten erhältlich sind, wobei der Hydrolysegrad z.B. 5 bis 100 mol-%, vorzugsweise 70 bis 100 mol-% beträgt. Die Hydrolyse der oben beschriebenen Polymerisate erfolgt nach bekannten Verfahren durch Einwirkung von Säuren, Basen oder Enzymen. Bei Verwendung von Säuren als Hydrolysemittel liegen die Vinylamineinheiten der Polymerisate als Ammoniumsalz vor, während bei der Hydrolyse mit Basen freie Aminogruppen entstehen.

Die Homopolymerisate der N-Vinylcarbonsäureamide und ihre Copolymerisate können zu 5 bis 100, vorzugsweise 70 bis 100 mol-% hydrolysiert sein. In den meisten Fällen beträgt der Hydrolysegrad der Homo- und Copolymerisate 80 bis 95 mol-%. Der Hydrolysegrad der Homopolymerisate ist gleichbedeutend mit dem Gehalt der Polymerisate an Vinylamineinheiten. Bei Copolymerisaten, die Vinylester einpolymerisiert enthalten, kann neben der Hydrolyse der N-Vinylformamideinheiten eine Hydrolyse der Estergruppen unter Bildung von Vinylalkoholeinheiten eintreten. Dies ist insbesondere dann der Fall, wenn man die Hydrolyse der Copolymerisate in Gegenwart von Natronlauge durchführt. Einpolymerisiertes Acrylnitril wird ebenfalls bei der Hydrolyse chemisch verändert. Hierbei entstehen beispielsweise Amidgruppen oder Carboxylgruppen. Die Vinylamineinheiten enthaltenden Homo- und Copolymeren können gegebenenfalls bis zu 20 mol-% an Amidineinheiten enthalten, die z.B. durch Reaktion von Ameisensäure mit zwei benachbarten Aminogruppen oder durch intramolekulare Reaktion einer Aminogruppe mit einer benachbarten Amidgruppe z.B. von einpolymerisiertem N-Vinylformamid entsteht. Die Molmassen der Vinylamineinheiten enthaltenden Polymerisate betragen z.B. 500 bis 10 Millionen, vorzugsweise 1000 bis 5 Millionen (bestimmt durch Lichtstreuung). Dieser Molmassenbereich entspricht beispielsweise K-Werten von 5 bis 300, vorzugsweise 10 bis 250 (bestimmt nach H. Fikentscher in 5 %iger wässriger Kochsalzlösung bei 25°C und einer Polymerkonzentration von 0,5 Gew.-%).

Die Vinylamineinheiten enthaltenden Polymeren werden vorzugsweise in salzfreier Form eingesetzt. Salzfreie wässrige Lösungen von Vinylamineinheiten enthaltenden Polymerisaten können beispielsweise aus den oben beschriebenen salzhaltigen Polymerlösungen mit Hilfe einer Ultrafiltration an geeigneten Membranen bei Trenngrenzen von beispielsweise 1000 bis 500 000 Dalton, vorzugsweise 10 000 bis 300 000 Dalton hergestellt werden. Auch die unten beschriebenen wässrigen Lösungen von Amino- und/oder Ammoniumgruppen enthaltenden anderen Polymeren können mit Hilfe einer Ultrafiltration in salzfreier Form gewonnen werden.

Auch Derivate von Vinylamineinheiten enthaltenden Polymeren können als basische Hydrogele bildende Polymere eingesetzt werden. So ist es beispielsweise möglich, aus den Vinylamineinheiten enthaltenden Polymeren durch Amidierung, Alkylierung, Sulfonamidbildung, Harnstoffbildung, Thioharnstoffbildung, Carbamatbildung, Acylierung, Carboximethylierung, Phosphonomethylierung oder Michaeladdition der Aminogruppen des Polymeren eine Vielzahl von geeigneten Hydrogelderivaten herzustellen. Von besonderem Interesse sind hierbei unvernetzte Polyvinylguanidine, die durch Reaktion von Vinylamineinheiten enthaltenden Polymeren, vorzugsweise Polyvinylaminen, mit Cyanamid ($R^1R^2N$-CN, wobei $R^1$ ,R2 = H, C1- bis C4-Alkyl, C3- bis C6-Cycloalkyl, Phenyl, Benzyl, alkylsubstituiertes Phenyl oder Naphthyl bedeuten) zugänglich sind, vgl. US-A-6,087,448, Spalte 3, Zeile 64 bis Spalte 5, Zeile 14.

Zu den Vinylamineinheiten enthaltenden Polymeren gehören auch hydrolysierte Pfropfpolymerisate von beispielsweise N-Vinylformamid auf Polyalkylenglkolen, Polyvinylacetat, Polyvinylalkolhol, Polyvinylformamiden, Polysacchariden wie Stärke, Oligosacchariden oder Monosacchariden. Die Pfropfpolymerisate sind dadurch erhältlich, daß man beispielsweise N-Vinylformamid in wäßrigem Medium in Gegenwart mindestens einer der genannten Pfropfgrundlagen gegebenenfalls zusammen mit copolymerisierbaren anderen Monomeren radikalisch polymerisiert und die aufgepfropften Vinylformamideinheiten anschließend in bekannten Weise zu Vinylamineinheiten hydrolysiert.

Für die Herstellung wasserabsorbierender basischer Polymere kommen auch Polymerisate von Dialkylaminoalkyl(meth)acrylamiden in Betracht. Geeignete Monomere für die Herstellung solcher Polymere sind beispielsweise Dimethylaminoethylacrylamid, Dimethylaminoethylmethacrylamid, Dimethylaminopropylacrylamid, Dimethylaminopropylmethacrylamid, Diethylaminoethylacrylamid, Diethylaminoethylmethacrylamid und Diethylaminopropylacrylamid. Diese Monomeren können in Form der freien Basen, der Salze mit anorganischen oder organischen Säuren oder in quaternisierter Form bei der Polymerisation eingesetzt werden. Sie können zu Homopolymerisaten oder zusammen mit anderen copolymerisierbaren Monomeren zu Copolymerisaten radikalisch polymerisiert werden. Die Polymerisate enthalten beispielsweise mindestens 30 Mol-%, vorzugsweise mindestens 70 Mol-.% der der genannten basischen Monomeren einpolymerisiert. Wasserabsorbierende basische Polymere auf Basis von Poly(Dimethylaminoalkylacrylamiden) sind aus der US-A-5,962,578 bekannt.

Weitere geeignete basische Polymere sind Polyethylenimine, die beispielsweise durch Polymerisation von Ethylenimin in wässriger Lösung in Gegenwart von säureabspaltenden Verbindungen, Säuren oder Lewis-Säuren als Katalysator hergestellbar sind. Polyethylenimine haben beispielsweise Molmassen bis zu 2 Millionen, vorzugsweise von 200 bis 1.000 000. Besonders bevorzugt werden Polyethylenimine mit Molmassen von 500 bis 750 000 eingesetzt. Die Polyethylenimine können gegebenenfalls modifiziert werden, z.B. alkoxyliert, alkyliert oder amidiert werden. Sie können au-ßerdem einer Michaeladdition oder einer Steckersynthese unterworfen werden. Die dabei erhältlichen Derivate von Polyethyleniminen sind ebenfalls als basische Polymere zur Herstellung von wasserabsorbierenden basischen Polymeren geeignet.

Außerdem kommen mit Ethylenimin gepfropfte Polyamidoamine in Betracht, die beispielsweise durch Kondensieren von Dicarbonsäuren mit Polyaminen und anschließendes Aufpfropfen von Ethylenimin erhältlich sind. Geeignete Polyamidoamine erhält man beispielsweise dadurch, daß man Dicarbonsäuren mit 4 bis 10 Kohlenstoffatomen mit Polyalkylenpolyaminen umsetzt, die 3 bis 10 basische Stickstoffatome im Molekül enthalten. Beispiele für Dicarbonsäuren sind Bernsteinsäure, Maleinsäure, Adipinsäure, Glutarsäure, Korksäure, Sebacinsäure oder Terephthalsäure. Bei der Herstellung der Polyamidoamine kann man auch Mischungen von Dicarbonsäuren einsetzen, ebenso Mischungen aus mehreren Polyalkylenpolyaminen. Geeignete Polyalkylenpolyamine sind beispielsweise Diethylentriamin, Triethylentetramin, Tetraethylenpentamin, Dipropylentriamin, Tripropylentetramin, Dihexamethylentriamin, Aminopropylethylendiamin und Bis-aminopropylethylendiamin. Die Dicarbonsäuren und Polyalkylenpolyamine werden zur Herstellung der Polyamidoamine auf höhere Temperaturen erhitzt, z.B. auf Temperaturen in dem Bereich von 120 bis 220, vorzugsweise 130 bis 180°C. Das bei der Kondensation entstehende Wasser wird aus dem System entfernt. Bei der Kondensation kann man gegebenenfalls auch Lactone oder Lactame von Carbonsäuren mit 4 bis 8 C-Atomen einsetzen. Pro Mol einer Dicarbonsäure verwendet man beispielsweise 0,8 bis 1,4 Mol eines Polyalkylenpolyamins. Diese Polyamidoamine werden mit Ethylenimin gepfropft. Die Pfropfreaktion wird beispielsweise in Gegenwart von Säuren oder Lewis-Säuren wie Schwefelsäure oder Bortrifluoridetheraten bei Temperaturen von beispielsweise 80 bis 100°C durchgeführt. Verbindungen dieser Art werden beispielsweise in der DE-B-24 34 816 beschrieben.

Auch die gegebenenfalls vernetzten Polyamidoamine, die gegebenenfalls noch zusätzlich vor der Vernetzung mit Ethylenimin gepfropft sind, kommen als basische Polymere in Betracht. Die vernetzten, mit Ethylenimin gepfropften Polyamidoamine sind wasserlöslich und haben z.B. ein mittleres Molgewicht von 3000 bis 2 Million Dalton. Übliche Vernetzer sind z.B. Epichlorhydrin oder Bischlorhydrinether von Alkylenglykolen und Polyalkylenglykolen.

Als basische Polymere kommen auch Polyallylamine in Betracht. Polymerisate dieser Art werden erhalten durch Homopolymerisation von Allylamin, vorzugsweise in mit Säuren neutralisierter Form oder durch Copolymerisieren von Allylamin mit anderen monoethylenisch ungesättigten Monomeren, die oben als Comonomere für N-Vinylcarbonsäureamide beschrieben sind.

Außerdem eignen sich wasserlösliche vernetzte Polyethylenimine, die durch Reaktion von Polyethyleniminen mit Vernetzern wie Epichlorhydrin oder Bischlorhydrinethern von Polyalkylenglykolen mit 2 bis 100 Ethylenoxid- und/oder Propylenoxid-Einheiten erhältlich sind und noch über freie primäre und/oder sekundäre Aminogruppen verfügen. Auch amidische Polyethylenimine sind geeignet, die beispielsweise durch Amidierung von Polyethyleniminen mit $C_1$- bis $C_{22}$-Monocarbonsäuren erhältlich sind. Weitere geeignete kationische Polymere sind alkylierte Polyethylenimine und alkoxylierte Polyethylenimine. Bei der Alkoxylierung verwendet man z.B. pro NH-Einheit im Polyethylenimin 1 bis 5 Ethylenoxid- bzw. Propylenoxideinheiten.

Die obengenannten basischen Polymerisate haben z.B. K-Werte von 8 bis 300, vorzugsweise 15 bis 180 (bestimmt nach H. Fikentscher in 5 %iger wässriger Kochsalzlösung bei 25 % und einer Polymerkonzentration von 0,5 Gew.-%). Bei einem pH-Wert von 4,5 haben sie beispielsweise eine Ladungsdichte von mindestens 1, vorzugsweise mindestens 4 mVal/g Polyelektrolyt.

Bevorzugt in Betracht kommende basische Polymere sind Vinylamineinheiten enthaltende Polymere, Polyvinylguanidine und Polyethylenimine. Beispiele hierfür sind:

Vinylamin-Homopolymere, 10 bis 100 % hydrolysierte Polyvinylformamide, partiell oder vollständig hydrolysierte Copolymerisate aus Vinylformamid und Vinylacetat, Vinylalkohol, Vinylpyrrolidon oder Acrylamid jeweils mit Molmassen von 3.000 - 2.000 000 sowie

Polyethylenimine, vernetzte Polyethylenimine oder amidierte Polyethylenimine, die jeweils Molmassen von 500 bis 3.000.000 haben. Der Polymergehalt der wäßrigen Lösung beträgt beispielsweise 1 bis 60, vorzugsweise 2 bis 15 und meistens 5 bis 10 Gew.-%.

Vernetzer

Um aus den oben beschriebenen basischen Polymeren wasserabsorbierende basische Polymere zu erhalten, setzt man sie mit mindestens einem Vernetzer um. Die basischen Polymeren sind meistens wasserlöslich bzw. leicht in Wasser dispergierbar. Die Vernetzung erfolgt daher hauptsächlich in wäßrigem Medium. Bevorzugt werden wäßrige Lösungen von basischen Polymeren eingesetzt, die beispielsweise mit Hilfe einer Ultrafiltration entsalzt sind bzw. deren Gehalt an Neutralsalzen unter 1 bzw. unter 0,5 Gew.-% liegt. Die Vernetzer haben mindestens zwei reaktive Gruppen, die mit den Aminogruppen der basischen Polymeren reagieren und dabei unlösliche Produkte bilden, die wasserabsorbierende Polymere darstellen. Pro 1 Gewichtsteil eines basischen Polymeren setzt man beispielsweise 0,1 bis 50, vorzugsweise 1 bis 5 Gew.-Teile und insbesondere 1,5 bis 3 Gew.-Teile eines Vernetzers ein. Geeignete Vernetzer werden in der WO-A-00/63295, Seite 14, Zeile 43 bis Seite 21, Zeile 5 beschrieben.

Geeignete bi- oder polyfunktionelle Vernetzer sind beispielsweise

(1) Di- und Polyglycidylverbindungen
(2) Di- und Polyhalogenverbindungen
(3) Verbindungen mit zwei oder mehr Isocyanatgruppen, die blokkiert sein können
(4) Polyaziridine
(5) Kohlensäurederivate
(6) Verbindungen mit zwei oder mehreren aktivierten Doppelbindungen, die eine Michael-Addition eingehen können
(7) Di- und Polycarbonsäuren sowie deren Säurederivate
(8) monoethylenisch ungesättigte Carbonsäuren, deren Ester, Amide und Anhydride
(9) Di- und Polyaldehyde und Di- und Polyketone.

Bevorzugte Vernetzer (1) sind beispielsweise die in der US-A-4 144 123 beschriebenen Bischlorhydrinethern von Polyalkylenglykolen. Weiterhin seien Phosphorsäurediglycidylether und Ethylenglykoldiglycidylether genannt.

Weitere Vernetzer sind die Umsetzungsprodukte von mindestens dreiwertigen Alkoholen mit Epichlorhydrin zu Reaktionsprodukten, die mindestens zwei Chlorhydrin-Einheiten aufweisen, z.B. verwendet man als mehrwertige Alkohole Glycerin, ethoxylierte oder propoxylierte Glycerine, Polyglycerine mit 2 bis 15 Glycerin-Einheiten im Molekül sowie gegebenenfalls ethoxylierte und/oder propoxylierte Polyglycerine. Vernetzer dieser Art sind beispielsweise aus der DE-A-2 916 356 bekannt.

Geeignete Vernetzer (2) sind $\alpha,\omega$- oder vicinale Dichloralkane, beipielsweise 1,2 Dichlorethan, 1,2 Dichlorpropan, 1,3-Dichlorbutan und 1,6-Dichlorhexan.

Ferner sind aus der EP-A-0 025 515 $\alpha,\omega$-Dichlorpolyalkylenglykole die vorzugsweise 1 - 100, insbesondere 1 - 100 Ethylenoxideinheiten haben, als Vernetzer bekannt.

Außerdem eignen sich Vernetzer (3), die blockierte IsocyanatGruppen enthalten, z.B. Trimethylhexamethylendiisocyanat blokkiert mit 2,2,6,6-Tetramethylpiperidin-4-on. Solche Vernetzer sind bekannt, vgl. beispielsweise aus DE-A-4 028 285.

Bevorzugt sind ferner Aziridin-Einheiten enthaltende Vernetzer (4) auf Basis von Polyethern oder substituierten Kohlenwasserstoffen, z.B. 1,6-Bis-N-aziridinomethan, vgl. US-A-3 977 923. In diese Vernetzerklasse fallen weiterhin mindestens zwei Aziridinogruppen enthaltende Umsetzungsprodukte von Dicarbonsäureestern mit Ethylenimin sowie Mischungen der genannten Vernetzer.

Als halogenfreie Vernetzer der Gruppe (4) kommen Reaktionsprodukte in Betracht, die durch Umsetzung von Dicarbonsäureestern, die mit einwertigen Alkoholen mit 1 bis 5 Kohlenstoffatomen vollständig verestert sind, mit Ethylenimin hergestellt werden. Geeignete Dicarbonsäureester sind beispielsweise Oxalsäuredimethylester, Oxalsäurediethylester, Bernsteinsäuredimethylester, Bernsteinsäurediethylester, Adipinsäuredimethylester, Adipinsäurediethylester und Glutarsäuredimethylester. So erhält man beispielsweise bei der Umsetzung von Diethyloxalat mit Ethylenimin Bis-[$\beta$-(1-Aziridino)ethyl]oxalsäureamid. Die Dicarbonsäureester werden mit Ethylenimin beispielsweise im Molverhältnis von 1 zu mindestens 4 umgesetzt. Reaktive Gruppen dieser Vernetzer sind die endständigen Aziridingruppen. Diese Vernetzer können beispielsweise mit Hilfe der Formel charakterisiert werden:

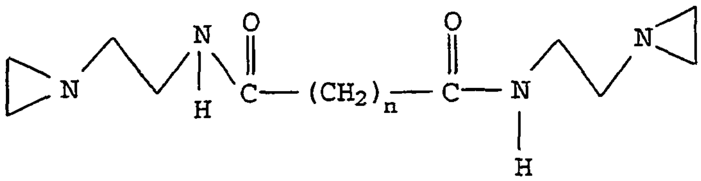

worin n = 0 bis 22 bedeutet.

Als Vernetzer (5) seien Ethylencarbonat, Propylencarbonat, Harnstoff, Thioharnstoff, Guanidin, Dicyandiamid oder 2-Oxazolidinon und dessen Derivate beispielhaft genannt. Aus dieser Gruppe von Monomeren wird vorzugsweise Propylencarbonat, Harnstoff und Guanidin verwendet.

Vernetzer (6) sind Umsetzungsprodukte von Polyetherdiaminen, Alkylendiaminen, Polyalkylenpolyaminen, Alkylenglykolen, Polyalkylenglykolen oder deren Gemischen mit monoethylenisch ungesättigten Carbonsäuren, Estern, Amiden oder Anhydriden monoethylenisch ungesättigter Carbonsäuren, wobei die Umsetzungsprodukte mindestens zwei ethylenisch ungesättigte Doppelbindungen, Carbonsäureamid-, Carboxyl- oder Estergruppen als funktionelle Gruppen aufweisen, sowie Methylenbisacrylamid und Divinylsulfon.

Vernetzer (6) sind beispielsweise Umsetzungsprodukte von Polyetherdiaminen mit bevorzugt 2 bis 50 Alkylenoxideinheiten, Alkylendiaminen wie Ethylendiamin, Propylendiamin, 1,4-Diaminobutan und 1,6-Diaminohexan, Polyalkylenpolyaminen mit Molekulargewichten < 5000 z.B. Diethylentriamin, Triethylentetramin, Dipropylentriamin, Tripropylentetramin, Dihexamethylentriamin und Aminopropylethylendiamin, Alkylenglykolen, Polyalkylenglykolen oder deren Gemischen mit

- monoethylenisch ungesättigten Carbonsäuren,
- Estern monoethylenisch ungesättigter Carbonsäuren,
- Amiden monoethylenisch ungesättigter Carbonsäuren und
- Anhydriden monoethylenisch ungesättigter Carbonsäuren.

Diese Umsetzungsprodukte sowie ihre Herstellung werden in der EP-A-873 371 beschrieben und seien als Vernetzer ausdrücklich erwähnt.

Besonders bevorzugt in Betracht kommende Vernetzer sind die hierin erwähnten Umsetzungsprodukte von Maleinsäureanhydrid mit $\alpha,\omega$-Polyetherdiaminen einer Molmasse von 400 bis 5000, die Umsetzungsprodukte von Polyethyleniminen einer Molmasse von 129 bis 50000 mit Maleinsäureanhydrid sowie die Umsetzungsprodukte von Ethylendiamin oder Triethylentetramin mit Maleinsäureanhydrid im Molverhältnis von 1: mindestens 2.

Als Vernetzer (6) verwendet man vorzugsweise die Verbindungen der Formel

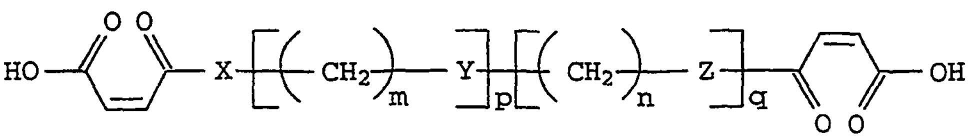

in der X, Y, Z = O, NH

und Y zusätzlich noch $CH_2$

m, n = 0 - 4

p, q = 0 - 45000

bedeuten,

die durch Umsetzung von Polyetherdiaminen, Ethylendiamin oder Polyalkylenpolyaminen mit Maleinsäureanhydrid erhältlich sind.

Weitere halogenfreie Vernetzer der Gruppe (7) sind mindestens zweibasische gesättigte Carbonsäuren wie Dicarbonsäuren sowie die davon abgeleiteten Salze, Diester und Diamide. Diese Verbindungen können beispielsweise mit Hilfe der Formel

$$X\text{-}CO\text{-}(CH_2)_n\text{-}CO\text{-}X$$

in der X= OH, $OR^1$, $N(R^2)_2$

$R^1$= $C_1$- bis $C_{22}$-Alkyl,

$R^2$= H, $C_1$-$C_{22}$-Alkyl und

n= 0 bis 22

charakterisiert werden. Außer den Dicarbonsäuren der obengenannten Formel eignen sich beispielsweise monoethylenisch ungesättigte Dicarbonsäuren wie Maleinsäure oder Itaconsäure. Die Ester der in Betracht kommenden Dicarbonsäuren leiten sich vorzugsweise von Alkoholen mit 1 bis 4 Kohlenstoffatomen ab. Geeignete Dicarbonsäureester sind beispielsweise Oxalsäuredimethylester, Oxalsäurediethylester, Oxalsäurediisopropylester, Bernsteinsäuredimethylester, Bernsteinsäuredieethylester, Bernsteinsäurediisopropylester, Bernsteinsäuredi-n-propylester, Bernsteinsäurediisobutylester, Adipinsäuredimethylester, Adipinsäurediethylester und Adipinsäurediisopropylester oder mindestens 2 Estergruppen enthaltende Michael-Additionsprodukte aus Polyetherdiaminen, Polyalkylenpolyaminen oder Ethylendiamin und Estern der Acrylsäure oder Methacrylsäure mit jeweils einwertigen 1 bis 4 C-Atome enthaltenden Alkoholen. Geeignete Ester von ethylenisch ungesättigten Dicarbonsäuren sind beispielsweise Maleinsäuredimethylester, Maleinsäurediethylester, Maleinsäurediisopropylester, Itaconsäuredimethylester und Itaconsäurediisopropylester. Außerdem kommen substituierte Dicarbonsäuren und ihre Ester wie Weinsäure (D,L-Form und als Racemat) sowie Weinsäureester wie Weinsäuredimethylester und Weinsäurediethylester in Betracht.

Geeignete Dicarbonsäureanhydride sind beispielsweise Maleinsäureanhydrid, Itaconsäureanhydrid und Bernsteinsäureanhydrid. Weiterhin sind als Vernetzer (7) Maleinsäuredimethylester, Maleinsäurediethylester und Maleinsäure beispielsweise geeignet. Die Vernetzung von Aminogruppen enthaltenden Verbindungen mit den vorstehend genannten Vernetzern erfolgt unter Bildung von Amidgruppen bzw. bei Amiden wie Adipinsäurediamid durch Umamidierung. Maleinsäureester, monoethylenisch ungesättigte Dicarbonsäuren sowie deren Anhydride können sowohl durch Bildung von Carbonsäureamidgruppen als auch durch Addition von NH-Gruppen der zu vernetzenden Komponente (z.B. Polyamidoaminen) nach Art einer Michael-Addition eine Vernetzung bewirken.

Zu den mindestens zweibasischen gesättigten Carbonsäuren der Vernetzerklasse (7) gehören beispielsweise Tri- und Tetracarbonsäuren wie Citronensäure, Propantricarbonsäure, Nitrilotriessigsäure, Ethylendiamintetraessigsäure, Butantetracarbonsäure und Diethylentriaminpentaessigsäure. Als Vernetzer der Gruppe (7) kommen außerdem die von vorstehend genannten Carbonsäuren abgeleiteten Salze, Ester, Amide und Anhydride z.B. Weinsäuredimethylester, Weinsäurediethylester, Adipinsäuredimethylester, Adipinsäurediethylester in Betracht.

Geeignete Vernetzer der Gruppe (7) sind außerdem Polycarbonsäuren, die durch Polymerisieren von monoethylenisch ungesättigten Carbonsäuren, Anhydriden, Estern oder Amiden erhältlich sind. Als monethylenisch ungesättigte Carbonsäuren kommen z.B. Acrylsäure, Methacrylsäure, Fumarsäure, Maleinsäure und/oder Itaconsäure in Betracht. So eignen sich als Vernetzer z.B. Polyacrylsäuren, Copolymerisate aus Acrylsäure und Methacrylsäure oder Copolymerisate aus Acrylsäure und Maleinsäure. Als Comonomere seine Vinylether, Vinylformiat, Vinylacetat und Vinyllactam beispielhaft genannt.

Weitere geeignete Vernetzer (7) werden z.B. durch radikalische Polymerisation von Anhydriden wie Maleinsäureanhydrid in einem inerten Lösemittel wie Toluol, Xylol, Ethylbenzol, Isopropylbenzol oder Lösemittelgemischen hergestellt. Außer den Homopolymerisaten kommen Copolymerisate von Maleinsäureanhydrid in Betracht, z.B. Copolymerisate aus Acrylsäure und Maleinsäureanhydrid oder Copolymerisate aus Maleinsäureanhydrid und einem $C_2$- bis $C_{30}$-Olefin.

Bevorzugte Vernetzer (7) sind beispielsweise Copolymerisate aus Maleinsäureanhydrid und Isobuten oder Copolymerisate aus Maleinsäureanhydrid und Diisobuten. Die Anhydridgruppen enthaltenden Copolymerisate können gegebenenfalls durch Umsetzung mit $C_1$- bis $C_{20}$-Alkoholen oder Ammoniak oder Aminen modifiziert sein und in dieser Form als Vernetzer eingesetzt werden.

Bevorzugte polymere Vernetzer (7) sind beispielsweise Copolymere aus Acrylamid und Acrylestern, wie beispielsweise Hydroxiethylacrylat oder Methylacrylat wobei das Mol-Verhältnis von Acrylamid und Acrylester von 90:10 bis zu 10:90 variieren kann. Neben diesen Copolymeren sind auch Terpolymere einsetzbar, wobei beispielsweise Kombinationen aus Acrylamid, Methacrylamid, Acrylestern bzw. Methacrylestern zur Verwendung kommen können.

Die Molmasse $M_w$ der als Vernetzer geeigneten Homo- und Copolymere beträgt z.B. bis zu 10000, vorzugsweise

500 bis 5000. Polymerisate der oben genannten Art werden z.B. beschrieben in EP-A-0 276 464, US-A-3 810 834, GB-A-1 411 063 und US-A-4 818 795. Die mindestens zweibasischen gesättigten Carbonsäuren und die Polycarbonsäuren können auch in Form der Alkali- oder Ammoniumsalze als Vernetzer eingesetzt werden. Bevorzugt verwendet man dabei die Natriumsalze. Die Polycarbonsäuren können partiell, z.B. zu 10 bis 50 mol-% oder auch vollständig neutralisiert sein.

Geeignete halogenfreie Vernetzer der Gruppe (8) sind z.B. monoethylenisch ungesättigte Monocarbonsäuren wie Acrylsäure, Methacrylsäure und Crotonsäure sowie die davon abgeleiteten Amide, Ester und Anhydride. Die Ester können sich von Alkoholen mit 1 - 22, vorzugsweise 1 bis 18 C-Atomen ableiten. Die Amide sind vorzugsweise unsubstituiert, können jedoch einen $C_1$-$C_{22}$-Alkylrest als Substituent tragen.

Bevorzugt eingesetzte Vernetzer (8) sind Acrylsäure, Acrylsäuremethylester, Acrylsäureethylester, Acrylamid und Methacrylamid.

Geeignete halogenfreie Vernetzer der Gruppe (9) sind z.B. Dialdehyde oder deren Halb- oder Acetale als Vorstufen, wie beispielsweise Glyoxal, Methylglyoxal, Malondialdehyd, Succindialdehyd, Malein- und Fumarsäuredialdehyd, Weinsäuredialdehyd, Adipindialdehyd, 2-Oxi-adipindialdehyd, Furan-2,5-dipropionaldehyd, 2-Formyl-2,3-dihydropyran, Glutardialdehyd, Pimelinsäurealdehyd sowie aromatische Dialdehyde wie beispielsweise Terephthaldialdehyd, o-Phthaldialdehyd, Pyridin-2,6-dialdehyd oder Phenylglyoxal. Es können aber auch Homo- oder Copolymere des Acroleins des Methacroleins mit Molmassen von 114 bis ca. 10000 verwendet werden. Als Comonomere können prinzipiell alle wasserlöslichen zum Einsatz kommen wie beispielsweise Acrylamid, Vinylacetat und Acrylsäure. Ebenso als Vernetzer geeignet sind Aldehydstärken.

Geeignete halogenfreie Vernetzer der Gruppe (9) sind z.B. Diketone oder die entsprechenden Halb- oder Ketale als Vorstufen wie beispielsweise β-Diketone wie Acetylaceton oder Cycloalkan-1,n-dione wie beispielsweise Cyclopentan-1,3-dion und Cyclohexan-1,4-dion. Es können aber auch Homo- oder Copolymere des Methylvinylketons mit Molmassen von 140 bis ca. 15000 verwendet werden. Als Comonomere können prinzipiell alle wasserlöslichen Monomere zum Einsatz kommen wie beispielsweise Acrylamid, Vinylacetat und Acrylsäure.

Es ist selbstverständlich auch möglich Mischungen aus zwei oder mehreren Vernetzern zu verwenden.

Bevorzugt eingesetzte Vernetzer sind Glycidylether von Alkylenglykolen wie Ethylenglycol, Propylenglykol, Butandiol-1,4, Hexandiol-1,6 und Polyalkylenglykolen mit Molmassen bis 1500 sowie die vollständig mit Acrylsäure und/oder Methacrylsäure veresterten Anlagerungsprodukte von 1 bis 25 Mol, vorzugsweise 2 bis 15 Mol Ethylenoxid and 1 Mol Trimethylolpropan oder Pentaerythrit.

Tenside

Die polymerisierbaren bzw. vernetzbaren wäßrigen Mischungen enthalten als weitere Komponente 0,1 bis 20 Gew.-% mindestens eines Tensids. Die Tenside sind für die Herstellung und die Stabilisierung des Schaums von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additionsprodukte von 80 Mol Ethylenoxid an 1 Mol Talgfettalkohol. Weitere handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere handelsübliche nichtionische Tenside werden durch Ethoxylierung von Rizinusöl erhalten. Pro Mol Rizinusöl werden beispielsweise 12 bis 80 Mol Ethylenoxid angelagert. Weitere handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$-Oxoalkohols, oder die Umsetzungsprodukte von 7 bis 8 Mol Ethylenoxid an 1 Mol eines $C_{13}/C_{15}$-Oxoalkohols. Weitere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines $C_{12}$- bis $C_{18}$-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten $C_{13}/C_{15}$-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche anionische Tenside. Weitere geeignete anionische Tenside sind Schwefelsäurehalbester von $C_{13}/C_{15}$-oxoalkoholen, Paraffinsulfonsäuren wie $C_{15}$-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure sowie Fettalkoholphosphate wie $C_{15}/C_{18}$-Fettalkoholphosphat. Die polymerisierbare wäßrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt der wäßrigen Mischung beträgt vorzugsweise 0,5 bis 10 Gew.-%. In den meisten Fällen weisen die wäßrigen Mischungen einen Tensidgehalt von 1,5 bis 8 Gew.-% auf.

Lösevermittler

Die vernetzbaren wäßrigen Mischungen können gegebenenfalls als weitere Komponente mindestens einen Lösevermittler enthalten. Hierunter sollen mit Wasser mischbare organische Lösemittel verstanden werden, z.B. Dimethylsulfoxid, Dimethylformamid, N-Methylpyrrolidon, einwertige Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glycerinmonomethylether.

Die wäßrigen Mischungen enthalten 0 bis 50 Gew.-% mindestens eines Lösevermittlers. Falls Lösevermittler eingesetzt werden, beträgt ihr Gehalt in der wäßrigen Mischung vorzugsweise 1 bis 25 Gew.-%.

Verdicker, Schaumstabilisatoren, Füllstoffe, Fasern, Zellkeimbildner

Die vernetzbare wäßrige Mischung kann gegebenenfalls Verdicker, Schaumstabilisatoren, Füllstoffe, Fasern und/oder Zellkeimbildner enthalten. Verdicker werden beispielsweise zur Optimierung der Schaumstruktur und zur Verbesserung der Schaumstabilität eingesetzt. Man erreicht damit, daß der Schaum während der Polymerisation nur geringfügig schrumpft. Als Verdickungsmittel kommen alle hierfür bekannten natürlichen und synthetischen Polymeren in Betracht, die die Viskosität eines wäßrigen Systems stark erhöhen und nicht mit den Aminogruppen der basischen Polymeren reagieren. Hierbei kann es sich um wasserquellbare oder wasserlösliche synthetische und natürliche Polymere handeln. Eine ausführliche Übersicht über Verdicker findet man beispielsweise in den Veröffentlichungen von R.Y. Lochhead und W.R. Fron, Cosmetics & Toiletries, 108, 95-135 (Mai 1993) und M.T. Clarke, "Rheological Additives" in D. Laba (ed.) "Rheological Properties of Cosmetics and Toiletries", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polyethylenglykole oder Copolymerisate aus Ethylenglykol und Propylenglykol sowie hochmolekulare Polysaccharide wie Stärke, Guarkernmehl, Johannisbrotkernmehl oder Derivate von Naturstoffen wie Carboxymethylcellulose Hydroxyethylcellulose, Hydroxymethylcellulose, Hydroxypropylcellulose und Cellulose Mischether. Eine weitere Gruppe von Verdickern sind wasserunlösliche Produkte, wie feinteiliges Siliciumdioxid, Zeolithe, Bentonit, Cellulosepulver, oder andere feinteilige Pulver von vernetzten Polymerisaten. Die wäßrigen Mischungen können die Verdicker in Mengen bis zu 30 Gew.-% enthalten. Falls solche Verdickungsmittel überhaupt eingesetzt werden, sind sie in Mengen von 0,1, vorzugsweise 0,5 bis 20 Gew.-% in der wäßrigen Mischung enthalten.

Um die Schaumstruktur zu optimieren, kann man gegebenenfalls Kohlenwasserstoffe mit mindestens 5 C-Atomen im Molekül zu der wäßrigen Reaktionsmischung zusetzen. Geeignete Kohlenwasserstoffe sind beispielsweise Pentan, Cyclopentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wäßrigen Mischung während des Schäumens liegt. Die aliphatischen Kohlenwasserstoffe erhöhen die Standzeit der noch nicht polymerisierten geschäumten wäßrigen Reaktionsmischung. Dadurch wird das Handling der noch nicht polymerisierten Schäume erleichtert und die Prozeßsicherheit erhöht. Die Kohlenwasserstoffe wirken beispielsweise als Zellkeimbildner und stabilisieren gleichzeitig den bereits gebildeten Schaum. Darüber hinaus können sie beim Polymerisieren des Monomerschaums ein weiteres Schäumen der Mischung bewirken. Sie können dann auch 5die Funktion eines Treibmittels haben. Anstelle von Kohlenwasserstoffen oder in Mischung damit kann man auch gegebenenfalls chlorierte oder fluorierte Kohlenwasserstoffe als Zellkeimbildner und/oder Schaumstabilisator einsetzen, z.B. Dichlormethan, Trichlormethan, 1,2-Dichlorethan, Trichlorfluormethan oder 1,1,2-Trichlortrifluorethan. Falls Kohlenwasserstoffe eingesetzt werden, verwendet man sie beispielsweise in Mengen von 0,1 bis 20 Gew.-%, vorzugsweise 0,1 bis 10 Gew.-%, bezogen auf die polymerisierbare wäßrige Mischung.

Um die Eigenschaften der Schaumstoffe zu modifizieren, kann man der vernetzbaren wäßrigen Mischung einen oder mehrere Füllstoffe zusetzen, z.B. Kreide, Talkum, Clay, Titandioxid, Magnesiumoxid, Aluminiumoxid, Fällungskieselsäuren in hydrophilen oder hydrophoben Modifikationen, Dolomit und/oder Calciumsulfat. Die Teilchengröße der Füllstoffe beträgt beispielsweise 10 bis 1000 μm, vorzugsweise 50 bis 850 μm. Die Füllstoffe können in Mengen bis zu 30 Gew.-% in der vernetzbaren wäßrigen Mischung enthalten sein.

Die Eigenschaften der Schaumstoffe können gegebenenfalls auch mit Hilfe von Fasern modifiziert werden. Hierbei kann es sich um natürliche oder synthetische Fasern bzw. um Fasergemische handeln, z.B. Fasern aus Cellulose, Wolle, Polyethylen, Polypropylen, Polyestern oder Polyamiden. Falls Fasern eingesetzt werden, können sie beispielsweise in einer Menge bis zu 200 Gew.-%, vorzugsweise bis zu 25 Gew.-% in der wäßrigen Mischung vorhanden sein. Füllstoffe und Fasern können gegebenenfalls auch der bereits geschäumten Mischung zugesetzt werden. Die Mitverwendung von Fasern führt zu einer Erhöhung der Festigkeitseigenschaften wie Naßfestigkeit, des fertigen Schaumstoffs.

Wasserabsorbierende saure Polymere

Als wasserabsorbierende saure Polymere, die nachfolgend auch als saure Superabsorber bezeichnet werden, können sämtliche Hydrogele eingesetzt werden, die z.B. in der WO-A-00/63295, Seite 2, Zeile 27 bis Seite 9, Zeile 16 beschrieben sind. Hierbei handelt es sich im Wesentlichen um schwach vernetzte Polymere von sauren Monomeren, die in zumindest partiell neutralisierter Form ein hohes Wasseraufnahmevermögen besitzen. Beispiele für solche jeweils geringfügig vernetzten Polymerisate sind vernetzte Polyacrylsäuren, vernetzte, hydrolysierte Pfropfpolymere von Acrylnitril auf Stärke, vernetzte Pfropfpolymerisate von Acrylsäure auf Stärke, hydrolysierte, vernetzte Copolymere aus Vinylacetat und Acrylsäureestern, vernetzte Polyacrylamide, hydrolysierte, vernetzte Polyacrylamide, vernetzte Copolymerisate aus Ethylen und Maleinsäureanhydrid, vernetzte Copolymerisate aus Isobutylen und Maleinsäureandhydrid, vernetzte Polyvinylsulfonsäuren, vernetzte Polyvinylphosphonsäuren und vernetztes sulfoniertes Poylstyrol. Die genannten sauren Superabsorber können entweder allein oder in Mischung miteinander der vernetzbaren wäßrigen Mischung zugesetzt werden. Vorzugsweise verwendet man als saure Superabsorber teilchenförmige Polymerisate von neutralisierten, geringfügig vernetzten Polyacrylsäuren. Die Neutralisation der Säuregruppen der sauren Superabsorber erfolgt vorzugsweise mit Natronlauge, Natriumhydrogencarbonat oder Natriumcarbonat. Die Neutralisation kann jedoch auch mit Kalilauge, Ammoniak, Aminen oder Alkanolaminen wie Ethanolamin, Diethanolamin oder Triethanolamin vorgenommen werden.

Die wasserabsorbierenden sauren Polymeren werden in partikulärer Form zu der vernetzbaren Mischung oder vorzugsweise zu einer bereits geschäumten vernetzbaren Mischung gegeben. Die Teilchen können in fester Form oder in geschäumter Form verwendet werden. Der mittlere Teilchendurchmesser nach dem Gewichtsmittel beträgt beispielsweise 10 bis 2000 μm, vorzugsweise 100 bis 850 μm und liegt meistens in dem Bereich von 150 bis 450 μm. Superabsorber mit den entsprechenden Teilchengrößen können beispielsweise durch Zerkleinern, z.B. durch Mahlen von grobkörnigen, festen Superabsorbern oder von geschäumten Superabsorbern hergestellt werden. Die Dichte der geschäumten, sauren Superabsorber beträgt beispielsweise 0,01 bis 0,9 g/cm3, vorzugsweise 0,05 bis 0,7 g/cm3. Die Oberfläche der partikulären Superabsorber kann gegebenenfals nachvernetzt sein. Vorzugsweise setzt man saure Superabsorber ein, deren Oberfläche nicht nachvernetzt ist.

Saure Superabsorber sind aus den obengenannten Literaturstellen bekannt, vgl. insbesondere WO-A-00/63295, Seite 6, Zeile 36 bis Seite 7, Zeile 44. Zur Oberflächennachvernetzung werden beispielsweise Teilchen aus geringfügig vernetzten Polyacrylsäuren mit Verbindungen umgesetzt, die mindestens zwei gegenüber Carboxylgruppen reaktive Gruppen aufweisen. Hierbei handelt es sich um typische Vernetzer, die oben unter (b) angegeben sind. Von besonderem Interesse für die Anwendung als Vernetzer sind beispielsweise mehrwertige Alkohole wie Propylenglykol, Butandiol-1,4 oder Hexandiol-1,6 und Glycidylether von Ethylenglykol und Polyethylenglykolen mit Molmassen von 200 bis 1500, vorzugsweise 300 bis 400, und vollständig mit Acrylsäure oder Methacrylsäure veresterte Umsetzungsprodukte von Trimethylolpropan, von Umsetzungsprodukten aus Trimethylolpropan und Ethylenoxid im Molverhältnis 1 : 1 bis 25, vorzugsweise 1 : 3 bis 15 sowie von Umsetzungsprodukten von Pentaerythrit mit Ethylenoxid im Molverhältnis 1 : 30, vorzugsweise 1 : 4 bis 20. Die Nachvernetzung der Oberfläche der anionischen Superabsorberteilchen wird beispielsweise bei Temperaturen bis zu 220°C, z.B. vorzugsweise bei 120 bis 190°C durchgeführt.

Als wasserabsorbierende saure Polymere setzt man Superabsorber in Form von Teilchen mit den oben angegebenen Teilchengrößen ein. Sofern man in die vernetzbare wäßrige Mischung wasserabsorbierende saure Polymere einarbeitet, so enthält die Polymermischung z.B. 10 bis 90, vorzugsweise 30 bis 70 Gew.-% eines wasserabsorbierenden sauren Polymers. Meistens enthält die Mischung aus geschäumtem basischen Hydrogel und dem gegebenenfalls geschäumten sauren Hydrogel 40 bis 60 Gew.-% des sauren Superabsorbers.

Um Schäume herzustellen, die auch gegenüber salzhaltigen wäßrigen Lösungen ein hohes Absorptionsvermögen aufweisen, setzt man die basischen und die sauren Superabsorber vorzugsweise in nichtneutralisierter Form ein. Der Neutralisationsgrad der sauren wasserabsorbierenden Polymeren beträgt beispielsweise 0 bis 100, vorzugsweise 0 bis 75 und meistens 0 bis 50 Mol-%. Die wasserabsorbierenden basischen Polymeren haben in Form der freien

Basen eine höhere Aufnahmekapazität für salzhaltige wäßrige Lösungen und insbesondere saure wäßrige Lösungen als in der mit Säuren neutralisierten Form. Wenn basische Polymere als alleinige wasserabsorbierende Polymere eingesetzt werden, so beträgt der Neutralisationsgrad beispielsweise 0 bis 100. Vorzugsweise 0 bis 60 Mol.-%.

Superabsorbierende Fasern und Fruchtfasern

Erfindungsgemäß enthalten die Schaumstoffe superabsorbierende Fasern, die vorzugsweise der wäßrigen polymerisierbaren Lösung vor dem Schäumen oder dem Schaum zugegeben werden. Superabsorbierende Fasern sind aus den zum Stand der Technik angegebenen Literaturstellen EP-B-0 264 208, EP-B-0 272 072, EP-B-0 436 514 und US-A-4,813,945 bekannt. Hierbei handelt es sich vorzugsweise um Fasern aus einem hydrolysierten und anschließend vernetzten Copolymerisat aus Isobuten und Maleinsäureanhydrid. Anstelle von Isobuten können die Copolymeren auch andere 1-Olefine wie Ethylen, Propylen, Diisobutylen oder Styrol einpolymerisiert enthalten. Die genannten Olefine und Styrol sind leicht mit Maleinsäureanhydrid copolymerisierbar. Die Copolymeren werden in wäßrigem Medium hydrolysiert, zu beispielsweise 20 bis 80 Mol.-% mit Natronlauge oder Kalilauge neutralisiert, mit Vernetzern gemischt, die mit den Carboxylgruppen der Copolymerisate reagieren können (z.B. mehrwertige Alkohole, mehrwertige Amine oder Aminoalkohole) und nach weitgehendem Entfernen des Wassers zu Fasern versponnen. Die Fasern werden durch Erhitzen auf Temperaturen von beispielsweise 170 bis 240°C vernetzt, wodurch sie zu Superabsorbern werden. Der Durchmesser der Fasern beträgt beispielsweise 5 bis 500 μm, vorzugsweise 10 bis 300 μm, die Länge der Fasern liegt beispielsweise in dem Bereich von 2 bis 60 mm, vorzugsweise 6 bis 12 mm. Die Fasern werden vorzugsweise der wäßrigen polymerisierbaren Mischung zugesetzt, können jedoch auch der geschäumten Mischung vor dem Aushärten durch Polymerisieren der Monomeren oder durch Vernetzen der basischen Polymeren zugegeben werden.

Außer synthetischen superabsorbierenden Fasern können auch natürliche Fasern verwendet werden. Beispiele für solche Fasern sind Fruchtfasern wie Apfelfasern, Orangenfasern, Tomatenfasern, Weizenfasern und/oder Haferfasern. Solche Fasern sind im Handel erhältlich. Sie werden beispielsweise von der Firma J. Rettenmaier & Söhne GmbH & Co., Faserstoff-Werke, D-73494 Rosenberg, unter der Bezeichnung Vitacel® angeboten. Für die im Handel erhältlichen natürlichen Fasern der genannten Art werden folgende Faserlängen angegeben:

Apfelfasern <30 μm bis ca. 1000 μm
Orangenfasern <35 μm bis ca. 1000 μm
Tomatenfasern <200 μm bis ca. 2000 μm
Weizenfasern 30 μm bis 300 μm
Haferfasern 35 μm bis 300 μm.

Die superabsorbierenden Fasern und die Fruchtfasern werden beispielsweise in Mengen von 0,05 bis 10 Gew.-%, vorzugsweise 0,1 bis 5 Gew.-%, bezogen auf die polymerisierbare Mischung eingesetzt. Die superabsorbierenden synthetischen Fasern haben beispielsweise ein Wasseraufnahmevermögen (Free Swell Capacity) von mindestens 30 g/g, vorzugsweise mindestens 40 g/g.

Herstellung der Schäume

Die oben beschriebenen vernetzbaren wäßrigen Mischungen, die die Monomeren bzw. das basische Polymer, Vernetzer superabsorbierende Fasern und Tensid zwingend sowie gegebenenfalls mindestens eine weitere Komponente enthalten, werden zunächst geschäumt. Man kann beispielsweise ein inertes Gas unter einem Druck von z.B. 2 bis 400 bar in der vernetzbaren wäßrigen Mischung lösen und sie anschließend auf Atmosphärendruck entspannen. Beim Entspannen aus einer Düse entsteht ein fließfähiger Schaum. Man kann die vernetzbare wäßrige Mischung auch nach einer anderen Methode schäumen, indem man darin feine Blasen eines inerten Gases dispergiert. Das Schäumen der vernetzbaren wäßrigen Mischung kann im Labor beispielsweise dadurch erfolgen, daß man die wäßrige Mischung in einer Küchenmaschine, die mit einem Schneebesen ausgerüstet ist, schäumt. Die Schaumerzeugung wird vorzugsweise in einer Inertgasatmosphäre durchgeführt, z.B. in Stickstoff oder Edelgasen unter Normaldruck oder erhöhtem Druck, z.B. bis zu 25 bar und anschließendes Entspannen durchgeführt. Die Konsistenz der Schäume, die Größe der Gasblasen und die Verteilung der Gasblasen im Schaum kann beispielsweise durch die Auswahl der Tenside, Lösevermittler, Schaumstabilisatoren, Zellkeimbildner, Verdickungsmittel und Füllstoffe in einem weiten Bereich variiert werden. Dadurch kann man die Dichte, den Grad der Offenzelligkeit des Schaumstoffs und Wandstärke des Schaumstoffs leicht einstellen. Die wäßrige Mischung wird vorzugsweise bei Temperaturen geschäumt, die unterhalb des Siedepunkts der Bestandteile der wäßrigen Mischung liegen, z.B. bei Raumtemperatur bis zu 100°C, vorzugsweise bei 20 bis 50°C. Man kann jedoch auch bei Temperaturen oberhalb des Siedepunkts der Komponente mit dem niedrigsten Siedepunkt arbeiten, indem man die Mischung in einem druckdicht verschlossenen Behälter schäumt. Man erhält vernetzbare, schaumförmige Mischungen, die fließfähig und über einen längeren Zeitraum stabil sind. Die Dichte der geschäumten, vernetzbaren

Mischung beträgt bei einer Temperatur von 20°C beispielsweise 0,01 bis 0,9 g/cm3.

Vernetzen der geschäumten Mischung

In der zweiten Stufe des Verfahrens erfolgt die Polymerisation der Monomeren bzw. die Vernetzung des basischen Polymeren unter Bildung eines wasserabsorbierenden basischen Polymeren. Bei der Polymerisation werden z.B. mindestens zwei ethylenisch ungesättigte Doppelbindungen enthaltende Verbindungen als Vernetzer eingesetzt. Die Polymerisation wird in Gegenwart üblicher Radikale bildender Initiatoren durchgeführt. Man erhält dann vernetzte Polymere, die superabsorbierend sind.

Das ursprünglich wasserlösliche basische Polymer wird durch die Vernetzung wasserunlöslich. Man erhält ein Hydrogel eines basischen Polymeren. Die vernetzbaren schaumförmigen Mischungen werden beispielsweise in geeignete Formen transferiert und darin erhitzt, so daß die Monomeren polymerisieren bzw. die Vernetzer mit dem basischen Polymer reagieren. Das geschäumte Material kann beispielsweise in der gewünschten Stärke auf ein temporäres Trägermaterial, das vorteilhafterweise mit einer Antihaftbeschichtung versehen ist, aufgebracht werden. Man kann beispielsweise den Schaum auf eine Unterlage aufrakeln. Eine andere Möglichkeit besteht darin, die schaumförmige wäßrige Mischung in Formen einzufüllen, die ebenfalls antihaftbeschichtet sind.

Da die geschäumte wäßrige Mischung eine lange Standzeit aufweist, eignet sich diese Mischung auch für die Herstellung von Verbundmaterialien. Sie kann beispielsweise auf ein permanentes Trägermaterial aufgebracht werden, z.B. Folien aus Polymeren (z.B. Folien aus Polyethylen, Polypropylen oder Polyamid) oder Metallen wie Aluminium. Man kann die geschäumte wäßrige Mischung auch auf Vliese, Fluff, Tissues, Gewebe, natürliche oder synthetische Fasern, oder auf andere Schäume auftragen. Bei der Herstellung von Verbundmaterialien kann es unter Umständen vorteilhaft sein, den Schaum in Gestalt von bestimmten Strukturen oder in unterschiedlicher Schichtdicke auf ein Trägermaterial aufzubringen. Es ist jedoch auch möglich, den Schaum auf Fluff-Schichten oder Vliese aufzutragen und diese Materialien so zu imprägnieren, daß der Fluff nach der Vernetzung integraler Bestandteil des Schaums ist. Die in der ersten Verfahrensstufe erhältliche geschäumte wäßrige Mischung kann auch zu großen Blöcken geformt und vernetzt werden. Die Blöcke können nach der Vernetzung zu kleineren Formkörpern geschnitten oder gesägt werden. Man kann auch sandwichartige Strukturen herstellen, indem man eine geschäumte wäßrige Mischung auf eine Unterlage aufträgt, die schaumförmige Schicht mit einer Folie bzw. Vliesen, Tissues, Geweben, Fasern oder anderen Schäumen abdeckt und die sandwichartige Struktur durch Erhitzen vernetzt. Es ist jedoch auch möglich, vor oder nach dem vernetzen mindestens eine weitere Schicht aus einer geschäumten, vernetzbaren Schicht aufzutragen und gegebenenfalls mit einer weiteren Folie, Vliesen, Tissues, Geweben, Fasern oder anderen Materialien zu bedecken. Der Verbund wird dann in der zweiten Verfahrensstufe der Vernetzung unterworfen. Man kann jedoch auch sandwichartige Strukturen mit weiteren Schaumschichten gleicher oder unterschiedlicher Dichte herstellen.

Erfindungsgemäße Schaumschichten mit einer Schichtdicke von bis zu etwa 1 Millimeter stellt man beispielsweise durch einseitiges Erwärmen oder insbesondere durch einseitiges Bestrahlen der geschäumten polymerisieren bzw. vernetzbaren wäßrigen Mischung her. Falls dickere Schichten eines Schaums hergestellt werden sollen, z.B. Schäume mit Dicken von mehreren Zentimetern, ist die Erwärmung des vernetzbaren geschäumten Materials mit Hilfe von Mikrowellen besonders vorteilhaft, weil auf diesem Wege eine relativ gleichmäßige Erwärmung erreicht werden kann. Die Vernetzung erfolgt dabei beispielsweise bei Temperaturen von 20 bis 180°C, vorzugsweise in dem Bereich von 20 bis 100°C, insbesondere bei Temperaturen von 65 bis 80°C. Bei dickeren Schaumschichten, die vernetzt werden sollen, wird die geschäumte Mischung beidflächig mit Wärme behandelt, z.B. mit Hilfe einer Kontaktheizung oder durch Bestrahlung. Die Dichte des basischen schaumförmigen Hydrogels entspricht im Wesentlichen der Dichte der vernetzbaren wäßrigen Mischung. Man erhält somit Schaumstoffe aus wasserabsorbierenden basischen Polymeren mit einer Dichte von beispielsweise 0,01 bis 0,9 g/$cm^3$, vorzugsweise 0,1 bis 0,7 g/$cm^3$. Die schaumförmigen basischen Polymeren sind offenzellig. Der Anteil an offenen Zellen beträgt beispielsweise mindestens 80%, vorzugsweise liegt er oberhalb von 90%. Besonders bevorzugt sind Schäume mit einem offenzelligen Anteil von 100%. Der Anteil an offenen Zellen im Schaum wird beispielsweise mit Hilfe der Scanning Electron Microscopy bestimmt .

Bevorzugt sind Schaumstoffe, die dadurch erhältlich sind, daß man von einer polymerisierbaren wäßrigen Mischung ausgeht, die zu mindestens 50% mit Natronlauge oder Kalilauge neutralisierte Acrylsäure, einen mindestens zwei ethylenisch ungesättigte Doppelbindungen enthaltenden Vernetzer, einen Initiator, superabsorbierende Fasern aus einem hydrolysierten und anschließend vernetzten Copolymerisat aus Isobuten und Maleinsäureanhydrid und mindestens ein Tensid enthält. Weitere Beispiele für superabsorbierende Schaumstoffe sind dadurch erhältlich, daß man eine polymerisierbare wäßrige Mischung schäumt, die mindestens ein basisches Polymer aus der Gruppe von Vinylamineinheiten enthaltenden Polymeren, Vinylguanidineinheiten enthaltenden Polymeren, Dialkylaminoalkyl(meth)acrylamideinheiten enthaltenden Polymeren, Polyethyleniminen, mit Ethylenimin gepfropften Polyamidoaminen und Polydiallyldimethylammoniumchloriden enthält.

Schaumstoffe mit besonders hoher Wasseraufnahmekapazität und einem verbesserten Aufnahmevermögen für elektrolythaltige wäßrige Lösungen sind durch Vernetzen von geschäumten wäßrigen Mischungen basischer Polymerisate erhältlich, die, bezogen auf die Polymermischung, 10 bis 90 Gew.-% eines feinteiligen, wasserabsorbierenden, sauren Polymeren enthalten. Das saure Hydrogel kann als festes partikuläres Polymer oder als geschäumtes teilchenförmiges Polymer mit Teilchengrößpen von beispielsweise 10 bis 2000 μm in den erfindungsgemäßen Schaumstoffen vorliegen.

Nach dem Vernetzen der geschäumten Mischung oder während des Vernetzens erfolgt die Trocknung des schaumförmigen Hydrogels. Hierbei werden Wasser und andere flüchtige Bestandteile aus dem vernetzten schaumförmigen Hydrogel entfernt. Die Trocknung erfolgt vorzugsweise nach dem Vernetzen des schaumförmigen Hydrogels. Beispiele für geeignete Trocknungsverfahren sind thermische Konvektionstrocknung wie beispielsweise Horden-, Kammer-, Kanal-, Flachbahn-, Teller-, Drehtrommel-, Rieselschacht-, Siebband-, Strom-, Wirbelschicht-, Fließbett-, Schaufel- und Kugelbetttrocknung, thermische Kontakttrocknung wie Heizteller-, Walzen-, Band-, Siebtrommel-, Schnecken-, Taumel- und Kontaktscheibentrocknung, Strahlungstrocknung wie beispielsweise Infrarottrocknung, dielektrische Trocknung z.B. Mikrowellentrocknung und Gefriertrocknung. Um unerwünschte Zersetzungs- und Vernetzungsreaktionen zu vermeiden, kann es vorteilhaft sein, die Trocknung bei reduziertem Druck, unter einer Schutzgasatmosphäre und/oder unter schonenden thermischen Bedingungen, bei denen die Produkttemperatur 120°C, bevorzugt 100°C, nicht überschreitet, durchzuführen. Besonders geeignete Trocknungsverfahren stellen die (Vakuum)bandtrocknung und die Schaufeltrocknung dar.

Nach dem Trocknungsschritt enthält das schaumförmige Hydrogel meistens kein Wasser mehr. Der Wassergehalt des geschäumten Materials kann jedoch durch Befeuchten des Schaums mit Wasser oder Wasserdampf beliebig eingestellt werden. Meistens beträgt der Wassergehalt des schaumförmigen Gels 1 bis 60 Gew.-%, vorzugsweise 2 bis 10 Gew.-%. Mit Hilfe des Wassergehalts kann die Flexibilität des schaumförmigen Hydrogels eingestellt werden. Vollständig getrocknete schaumförmige Hydrogele sind hart und spröde, während geschäumte Materialien mit einem Wassergehalt von beispielsweise 5 bis 20 Gew.-% flexibel sind. Die geschäumten Hydrogele können entweder in Form von Folien oder Granulaten direkt verwendet werden oder man schneidet aus dickeren Schaum-Blöcken einzelne Platten oder Folien.

Die oben beschriebenen schaumförmigen Hydrogele können jedoch noch dahingehend modifiziert werden, daß man die Oberfläche der geschäumten Materialien nachvernetzt. Dadurch kann die Gelstabilität der Formkörper aus den geschäumten Hydrogelen verbessert werden. Um eine Oberflächennachvernetzung durchzuführen, behandelt man die Oberfläche der Formkörper aus den geschäumten Hydrogelen mit mindestens einem Vernetzungsmittel und erhitzt die so behandelten Formkörper auf eine Temperatur, bei der die Vernetzer mit den Hydrogelen reagieren. Geeignete Vernetzer sind oben beschrieben. Diese Verbindungen können ebenfalls für die Nachvernetzung der Oberfläche der schaumförmigen Hydrogele verwendet werden. Bevorzugt eingesetzte Vernetzer sind die oben bereits genannten Glycidylether und Ester der Acrylsäure und/oder Methacrylsäure mit den Umsetzungsprodukten aus 1 Mol Trimethylolpropan und 6 bis 15 Mol Ethylenoxid oder mehrwertige Alkohole, die z.B. zur Nachvernetzung von Carboxylgruppen enthaltenden schaumförmigen Superabsorbern verwendet werden.

Die Vernetzer für die Oberflächennachvernetzung werden vorzugsweise in Form einer wasserhaltigen Lösung auf die Schaumstoffoberfläche aufgebracht. Die wasserhaltige Lösung kann wassermischbare organische Lösemittel enthalten, z.B. Alkohole wie Methanol, Ethanol und/oder i-Propanol oder Ketone wie Aceton. Die Menge an Vernetzer, die auf die Oberfläche der schaumförmigen Hydrogele aufgetragen wird, beträgt beispielsweise 0,1 bis 5 Gew.-%, vorzugsweise 1 bis 2 Gew.-%. Die Oberflächennachvernetzung der schaumförmigen Hydrogele erfolgt durch Erhitzen der mit mindestens einem Vernetzer behandelten schaumförmigen Hydrogele bei einer Temperatur von beispielsweise 60 bis 120°C, vorzugsweise bei 70 bis 100°C. Nach der Oberflächenvernetzung kann der Wassergehalt der geschäumten, an der Oberfläche nachvernetzten Hydrogele ebenfalls auf Werte von 1 bis 60 Gew.-% eingestellt werden.

Die erfindungsgemäßen schaumförmigen Hydrogele, die gegebenenfalls an der Oberfläche nachvernetzt sind, können für alle Zwecke verwendet werden, für die beispielsweise die aus der EP-B-0 858 478 bekannten wasserabsorbierenden, schaumförmigen Hydrogele auf Basis von Säuregruppen enthaltenden Polymeren wie vernetzten Polyacrylaten, eingesetzt werden. Die erfindungsgemäßen schaumförmigen Hydrogele eignen sich beispielsweise für den Einsatz in Hygieneartikeln zur Absorption von Körperflüssigkeiten, in Verbandmaterial zur Abdeckung von Wunden, als Dichtungsmaterial, als Verpackungsmaterial, als Bodenverbesserungsmittel, als Bodenersatzstoff, zur Entwässerung von Schlämmen, zur Absorption saurer wäßriger Abfälle, zum Eindicken wäßriger Lacke bei der Entsorgung von Restmengen an Lacken, zur Entwässerung von wasserhaltigen Ölen oder Kohlenwasserstoffen oder als Material für Filter in Lüftungssystemen.

Von besonderer Bedeutung ist die Anwendung der erfindungsgemäßen schaumförmigen Hydrogele in Hygieneartikeln, wie Babywindeln, Damenbinden, Inkontinenzartikeln und in Verbandmaterial. Sie erfüllen beispielsweise in Hygieneartikeln mehrere Funktionen, nämlich Akquisition, Distribution und/oder Speicherung von Körperflüssigkeiten. Die Oberfläche der schaumförmigen Hydrogele kann gegebenenfalls durch Behandlung mit Tensiden oder unvernetzten Vinylamineinheiten enthaltenden Polymeren modifiziert werden. Man erzielt dadurch eine Verbesserung der Akquisition von Flüssigkeiten.

Schaumstoffschichten aus den erfindungsgemäß schaumförmigen Hydrogelen können beispielsweise in einer

Stärke von 1 bis 5 mm in einem der obengenannten Hygieneartikel als absorbierender Kern zwischen einer oberen flüssigkeitsdurchlässigen Abdeckung und einer unteren flüssigkeitsunduchlässigen Schicht aus einer Folie aus z.B. Polyethylen oder Polypropylen angeordnet sein. Die flüssigkeitsdurchlässige Schicht des Hygieneartikels steht bei der Anwendung in direktem Kontakt mit der Haut des Anwenders. Dieses Material besteht überlicherweise aus einem Vlies aus natürlichen Fasern wie Cellulosefasern oder Fluff. Gegebenenfalls ist oberhalb und/oder unterhalb des absorbierenden Kerns noch eine Tissueschicht angeordnet. Zwischen der untern Schicht des Hygieneartikels und dem absorbierenden Kern kann gegebenenfalls noch eine Speicherschicht aus einem herkömmlichen partikulären anionischen Superabsorber vorhanden sein. Wenn man die geschäumten basischen Hydrogele als absorbierenden Kern in Windeln einsetzt, so kann aufgrund der offenzelligen Struktur der geschäumten basischen Hydrogele die normalerweise in einzelnen Mengen auf einmal beaufschlagte Körperflüssigkeit zügig abgeführt werden.
Dadurch wird dem Anwender ein angenehmes Gefühl der Oberflächentrockenheit der Windel vermittelt.

Bestimmungsmethoden

Dichte

Jede geeignete gravimetrische Methode kann zur Dichtebestimmung des Mehrkomponenten-Schaumstoffsystems herangezogen werden. Ermittelt wird die Masse an festem Mehrkomponenten-Schaumstoffsystem pro Volumeneinheit Schaumstoffstruktur. Ein Verfahren zur Dichteermittlung des Mehrkomponenten-Schaumstoffsystems ist in der ASTM Methode Nr. D 3574-86, Test A, beschrieben. Diese Methode wurde ursprünglich zur Dichtebestimmung von Urethanschäumen entwickelt, kann aber auch zu diesem Zweck herangezogen werden. Danach wird bei einer vorkonditionierten Probe, wie in der Methode beschrieben, bei 22 +/- 2 °C deren Trockenmasse und Volumen ermittelt. Volumenbestimmungen größerer Probenabmessungen werden unter Normaldruck durchgeführt.

Free swell capacity (FSC)

Bei dieser Methode wird die freie Quellbarkeit des Mehrkomponenten-Schaumstoffsystems im Teebeutel bestimmt. Zur Bestimmung der FSC werden 0,2000 ± 0,0050 g getrockneter Schaumstoff in einem 60 x 85 mm großen Teebeutel eingeführt, der anschließend verschweißt wird. Der Teebeutel wird für 30 Minuten in einen Überschuß von Testlösung gegeben (mindestens 0,83 l Kochsalz-Lösung / 1 g Polymer). Der Teebeutel wird anschließend für 10 Minuten abtropfen gelassen, indem er an einer Ecke aufgehangen wird. Die Bestimmung der Flüssigkeitsmenge geschieht durch Auswägen des Teebeutels.
Als Testlösung wurde 0,9 Gew.%ige NaCl-Lösung eingesetzt.

Zentrifugenretentionskapazität (CRC = Centrifuge Retention Capacity)

Bei dieser Methode wird die freie Quellbarkeit des Mehrkomponenten-Schaumstoffsystems im Teebeutel bestimmt. Zur Bestimmung der CRC werden 0,2000 ± 0,0050 g getrockneter Mehrkomponenten-Schaumstoff in einem 60 x 85 mm großen Teebeutel eingeführt, der anschließend verschweißt wird. Der Teebeutel wird für 30 Minuten in einen Überschschuß von 0,9 Gew.%igen Kochsalzlösung gegeben (mindestens 0,83 l Kochsalz-Lösung / 1 g Polymer). Anschließend wird der Teebeutel 3 Minuten lang bei 250 G zentrifugiert. Die Bestimmung der Flüssigkeitsmenge geschieht durch Auswägen des zentrifugierten Teebeutels.
Als Testlösung wurde 0,9 Gew.%ige NaCl-Lösung eingesetzt.

Free Swell Rate (FSR)

Zur Bestimmung der Free Swell Rate werden 0,50 g ($W_H$) des Mehrkomponenten-Schaumstoffsystems auf dem Boden einer Plastikschale mit einem runden Boden von ca. 6 cm vorgelegt. Die Plastikschale hat eine Höhe von ca. 2,5 cm und besitzt eine quadratische Öffnung von ca. 7,5 cm x 7,5 cm. Mit Hilfe eines Trichters werden nun 10 g ($W_U$) einer 0,9% NaCl Lösung, in das Zentrum der Plastikschale zugegeben. Sobald die Flüssigkeit Kontakt mit dem Mehrkomponenten-Schaumstoffsystem hat, wird die Zeitmessung begonnen und erst dann gestoppt, wenn das Mehrkomponenten-Schaumstoffsystem die gesamte Flüssigkeit vollständig aufgenommen hat, d. h. bis keine freie Flüssigkeit mehr zu erkennen ist. Diese Zeit wird als $t_A$ notiert. Die Free Swell Rate berechnet sich dann gemäß

$$FSR = W_U / (W_H \times t_A).$$

K-Wert

Der K-Wert wurde nach H. Fikentscher, Cellulose-Chemie, Band 13, 52-63 und 71-74 (1932) in 5 gew.-%iger wäßriger Lösung bei pH 7, 25°C und einer Polymerkonzentration von 0,5 Gew.-% bestimmt.

Naßbruchwert (Wet Failure Value) von superabsorbierenden Schäumen (WFV)

Der Naßbruchwert ist diejenige Kraft, die erforderlich ist, um einen Testkörper aus einem vollständig gequollenen superabsorbierenden Schaum in der unten beschriebenen Vorrichtung kontrolliert zu zerstören. Der superabsorbierende Schaum wird in synthetischem Urin oder in 0,9 gew.-%iger wäßriger Kochsalzlösung solange gequollen, bis er keine Flüssigkeit mehr aufnimmt.

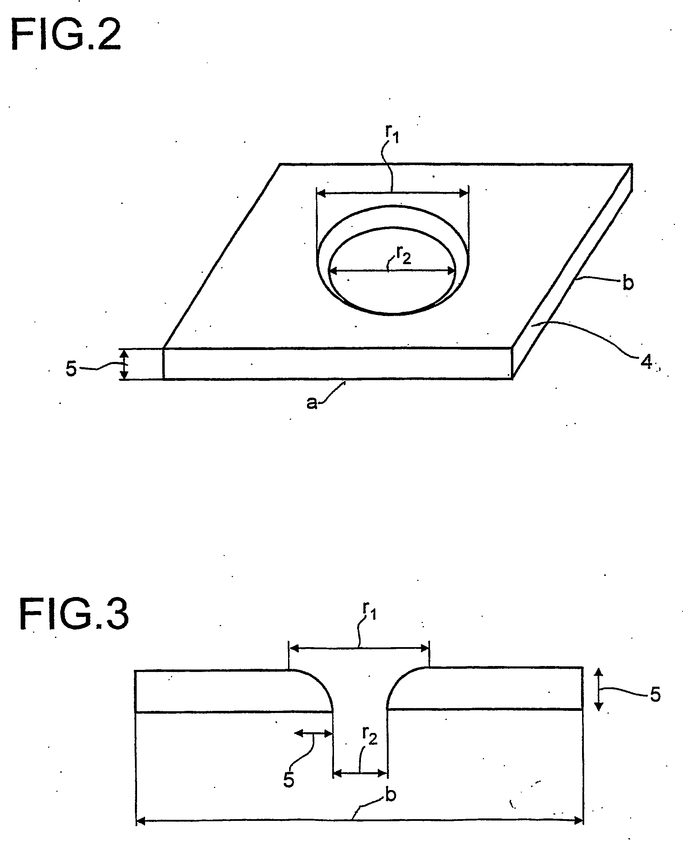

Die Messung des Naßbruchwertes erfolgt in einem handelsüblichen Texture Analyser (TA-XT2) der Firma Stable Micro Systems, Surrey, UK. Das Meßgerät ist in Figur 1 schematisch dargestellt. An einem Meßarm (1) ist eine Kugel (2) aus rostfreiem Stahl mit einem Durchmesser von 1 Zoll (2,54 cm) angebracht, die gegen eine zwischen zwei Metallplatten fixierte Probe des gequollenen superabsorbierenden Schaums (3) bewegbar ist. Beide Metallplatten weisen in der Mitte eine Bohrung mit einem Durchmesser (r1) = 5,1 cm und einem Durchmesser (r2) = 3,5 cm auf, vgl. Figur 2. Wie aus Figur 3 hervorgeht, weist die Seite der durchbohrten Platte mit dem Durchmesser (r1) eine Rundung auf, die einem Viertel Segment eines Kreises mit einem Durchmesser von 0,8 cm entspricht. Nur diese Seite der Platten kommt mit dem zu untersuchenden schaumförmigen Superabsorber in Kontakt. Die Rundung - vgl. Figur 3 - ist wichtig, damit der zu prüfende Schaum beim Testen nicht durch scharfkantige Ecken beschädigt wird. Die Oberfläche der Platten, die mit dem Schaum in Berührung kommt, ist aufgerauht, damit der Schaum während der Prüfung fixiert ist.

Die Platten haben jeweils eine Wandstärke von 0,8 cm und Kantenlängen a = 10 cm und b = 9 cm. Die Schaumprobe wird wie oben angegeben zwischen beiden Platten angeordnet. Die Belastung des Gerätes wird auf 5000 g eingestellt. Um den Naßbruchwert zu ermitteln, wird dann die mit dem Meßarm (1) verbundene Kugel (2) mit einer Geschwindigkeit von 0,5 mm/s gesenkt und die Kraft gemessen, die notwendig ist, um die Schaumprobe zu zerstören. Die maximale Distanz, die die Kugel (2) bei der Messung durchläuft, beträgt 30 mm. Die Kugel durchstößt dabei den Schaum, der sich zwischen den beiden Platten befindet. Die dafür erforderliche Kraft pro Fläche wird ermittelt und als WFV in g/mm2 angegeben.

Von jedem Schaum wurden 3 Proben vorbereitet und wie oben beschrieben gemessen. Hierbei ist es wichtig, daß die zu untersuchenden Schaumproben keine Löcher oder größere Lufteinschlüsse enthalten, weil dadurch die Meßergebnisse verfälscht werden.

Bestimmung der Dicke des gequollenen Schaums

Die Dicke des gequollenen Schaums wurde mit Hilfe eines Digimatic Dicken-Meßgerätes der Firma Mitutoyo ermittelt. Die Dicke eines Schaums wurde gemessen, sobald er vollständig gequollen war.

Querschnittsfläche (CSA)

Die Fläche des Querschnitts wird am vollständig gequollenen Schaum bestimmt, wobei man nur die Fläche berücksichtigt, die sich aus dem Durchmesser (r2 = 35 mm) und der Dicke des gequollenen Schaums im Gleichgewichtszustand (Te) nach folgender Formel ergibt:

$$CSA\ [mm^2] = 35 \times Te$$

Naßbruchpunkt

Hierunter wird die maximale Kraft (F) [g] verstanden, die im Texture Analyser durch das Peak-Maximum angezeigt wird und die notwendig ist, um die vollständig gequollene Schaumprobe im Texture Analyser gemäß Figur 1 zu zerstören.

Naßbruchwert

Der Naßbruchwert (WFV) ergibt sich aus der maximal notwendigen Kraft (F) für die Zerstörung der gequollenen Schaumprobe und der Querschnittsfläche des vollständig gequollenen Schaums gemäß

$$WFV\ [g/mm^2] = maximale\ Kraft/\ CSA$$

Superabsorbierende Fasern

Die in den Beispielen eingesetzten superabsorbierenden Fasern des Typs Fiberdri® von Camelot Technologies Limited, Canada, basieren auf vernetzten, hydrolysierten Copolymerisaten aus Isobuten und Maleinsäureanhydrid in teilweise mit Natronlauge neutralisierter Form.

Die in Beispielen außerdem verwendeten superabsorbierenden Fasern des Typs OASIS® von Technical Absorbents Limited, UK, basieren auf vernetzten Copolymerisaten aus Natriumacrylat, Hydroxypropylacrylat und Methylacrylat.

Sofern aus dem Zusammenhang nichts anderes hervorgeht, bedeuten die Prozentangaben in den Beispielen Gewichtsprozent.

Herstellung eines sauren teilchenförmigen wasserabsorbierenden Polymeren (SAP 1)

270g Acrylsäure wurden in ein Becherglas eingewogen. Man gab dann 1,155g Methylenbisacrylamid (MBA) als Vernetzer zu und rührte die Monomeren bis zur vollständigen Lösung. In einem separaten Gefäß wurden 810 g destilliertes Wasser eingewogen und zur Monomermischung gegeben. Die Lösung wurde gerührt, um die Mischung zu vervollständigen. Die wäßrige Monomerlösung wurde anschließend zum Abkühlen in einen Gefrierschrank aufbewahrt (etwa 1 Stunde).

Mit destilliertem Wasser stellte man eine 10%ige Natriumpersulfatlösung her, und gibt sie in einen gekühlten Polymerisationskessel. Als Initiatorsystem wurden 0,157 g 2-Hydroxy-2-Methyl-1-Phenyl-Propan-1-on (Darocur® 1173, Ciba, Photoinitiator) und 2,736 g der 10% Natriumpersulfatlösung zugesetzt. Nach einem abschließenden Vermischungsschritt erhielt man ein homogenes System, das so belassen wurde, bis es eine Temperatur von 10 °C erreicht hatte, bei der dann die Polymerisationsreaktion binnen 12 Minuten durch Bestrahlen mit einer UV Energie von $20mWcm^{-2}$ durchgeführt wurde. Man erhielt ein gelförmiges Polymerisat, das zerkleinert und bei 125 °C vollständig getrocknet wurde. Das erhaltene getrocknete Polymerisat wurde gemahlen und die Fraktion mit einer mittleren Teilchengröße von 150μm-450μm abgesiebt.

Beispiele

Beispiel 1

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37,3 %igen Natriumacrylatlösung in Wasser (0.54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 0.5gew% bez. Monomere (2,4g) superabsorbierenden Faser (Fiberdri P8/00 1231, ex Firma Camelot Technologies Limited, Canada). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azo-bis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Hönhle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 81,74 % |
| Neutralisationsgrad: | 60 mol% |
| Monomerschaumdichte: | 0,24 $gcm^{-3}$ |
| Polymerschaumdichte: | 0,20 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Beispiel 2

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37,3 %igen Natriumacrylatlösung in Wasser (0.54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 1.0 gew % bez. Monomere (4,8g) superabsorbierenden Faser (Fiberdri P8/00 1231, ex Firma Camelot Technologies Limited, Canada). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azo-bis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Hönhle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 82,13 % |
| Neutralisationsgrad: | 60 mol% |
| Monomerschaumdichte: | 0,28 $gcm^{-3}$ |
| Polymerschaumdichte: | 0,22 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Beispiel 3

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37,3 %igen Natriumacrylatlösung in Wasser (0,54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 3 gew % bez. Monomere (14,40g) superabsorbierenden Faser (Fiberdri P8/00 1231, ex Firma Camelot Technologies Limited, Canada). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azo-bis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 81,99 % |
| Neutralisationsgrad: | 60 mol% |
| Monomerschaumdichte: | 0,26 $gcm^{-3}$ |
| Polymerschaumdichte: | 0,21 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Beispiel 4

In einem Becherglas wurden mit Hilfe eines Magnetrührers die folgenden Komponenten vermischt.

| | |
|---|---|
| 348,55 g | Acrylsäure (4,84 mol) |
| 135,51 g | einer 37,3 %igen Natriumacrylatlösung in Wasser (0,54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 0,1 Gew-% (0,48 g), bezogen auf Monomere, der superabsorbierenden Faser (Fiberdri P8/00 1231, ex Firma Camelot Technologies Limited, Canada). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azobis(2-amidinopropan) dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 81,43 % |
| Neutralisationsgrad: | 60 mol % |
| Monomerschaumdichte: | 0,21 $gcm^{-3}$ |
| Polymerschaumdichte: | 0,19 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Beispiel 5

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37,3 %igen Natriumacrylatlösung in Wasser (0.54mol) |
| 28 g | Polyethylenglykoldiacrylat eines Polyethylenglycols der Molmasse 400 |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 1,0 Gew.-% (4,8 g), bezogen auf Monomere, der superabsorbierenden Faser (Fiberdri P8/00 1231, ex Firma Camelot Technologies Limited, Canada). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azobis(2-amidinopropan) dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 82.13 % |
| Neutralisationsgrad: | 60 mol% |
| Monomerschaumdichte: | 0,23 $gcm^{-3}$ |
| Polymerschaumdichte: | 0,20 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Beispiel 6

In einem Becherglas wurden mit Hilfe eines Magnetrührers die folgenden Komponenten vermischt.

| | |
|---|---|
| 348,55 g | Acrylsäure (4,84 mol) |
| 135,51 g | einer 37,3 %igen Natriumacrylatlösung in Wasser (0.54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 1 Gew.-%, bezogen auf Monomere (0,48g), superabsorbierenden Faser (OASIS ®, ex Technical Absorbents Limited, UK). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azobis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS -

Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 82,13 % |
| Neutralisationsgrad: | 60 mol % |
| Monomerschaumdichte: | 0,33 $gcm^{-3}$ |
| Polymerschaumdichte: | 0,29 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Beispiel 7

Zu 300g einer 10 %igen ultrafiltrierten wäßrigen Lösung von Polyvinylamin (PVAm) mit einem K-Wert von 90 fügte man 15g einer 5%igen wäßrigen Lösung eines handelsüblichen Tensids (Additionsprodukt von 80 Mol Ethylenoxid an 1 Mol eines C16/C18-Alkohol-gemisches) und 15g einer 5 %igen wäßrigen Lösung von Ethylenglykoldiglycidylether als Vernetzer.

Die vernetzbare Mischung wurden danach jeweils 1 Minute in Scherfeld eines Ultraturrax-Rührers geschäumt. Zu Proben der vernetzbaren wäßrigen Mischung fügte man dann 1 Gew.- % (0,3 g), bezogen auf PVAm, einer superabsorbierenden Faser (Fiberdri P8/00 1231, ex Firma Camelot Technologies Limited, Canada). Die Mischung wurde dann 1 Minute gerührt. Man erhielt eine homogene Mischung. Die so hergestellten vernetzbaren schaumförmigen Mischungen wurden dann jeweils auf eine Unterlage aus Teflon gegossen, die mit einer Umrandung aus Aluminium versehen war. Die Form, die die geschäumte vernetzbare Mischung enthielt, wurde über Nacht in einem Trockenschrank bei einer Temperatur von 70°C gelagert. Dabei wurde das erhaltene schaumförmige Hydrogel wurde anschließend auf einen Wassergehalt von 5 % eingestellt.

| | |
|---|---|
| Feststoffgehalt der Reaktionsmischung: | 10 % |
| Neutralisationsgrad: | 0 mol% |
| Polymerschaumdichte: | 0,18 $gcm^{-3}$ |
| Schaumstruktur: | homogen, vollständig offenzellig |

Weitere Eigenschaften des offenzelligen Schaums sind in Tabellen 1 und 2 angegeben.

Beispiel 8

Zu 300g einer 10 %igen ultrafiltrierten wäßrigen Lösung von Polyvinylamin mit einem K-Wert von 90 fügte man 15g einer 5%igen wäßrigen Lösung eines handelsüblichen Tensids (Additionsprodukt von 80 Mol Ethylenoxid an 1 Mol eines C16/C18-Alkoholgemisches) und 15g einer 5 %igen wäßrigen Lösung von Ethylenglykoldiglycidylether als Vernetzer.

Die vernetzbare Mischung wurden danach jeweils 1 Minute in Scherfeld eines Ultraturrax-Rührers geschäumt. Zu Proben der vernetzbaren wäßrigen Mischung fügte man zuerst 45g SAP 1 und dann 1 Gew.-% (0,3 g), bezogen auf PVAm, einer superabsorbierenden Faser (Fiberdri P8/00 1231, ex Firma Camelot Technologies Limited, Canada). Die Mischung wurde dann 1 Minute gerührt. Man erhielt eine homogene Mischung. Die so hergestellten vernetzbaren schaumförmigen Mischungen wurden dann jeweils auf eine Unterlage aus Teflon gegossen, die mit einer Umrandung aus Aluminium versehen war. Die Form, die die geschäumte vernetzbare Mischung enthielt, wurde über Nacht in einem Trockenschrank bei einer Temperatur von 70°C gelagert. Dabei wurde das erhaltene schaumförmige Hydrogel wurde anschließend auf einen Wassergehalt von 5 % eingestellt.

| | |
|---|---|
| Feststoffgehalt der Reaktionsmischung: | 22,5 % |
| Polymerschaumdichte: | 0,20 $gcm^{-3}$ |
| Schaumstruktur: | homogen, vollständig offenzellig |

Weitere Eigenschaften des offenzelligen Schaums sind in Tabellen 1 und 2 angegeben.

Vergleichsbeispiel 1

(Vergleich gemäß Beispiel 1 der WO-A-00/52087)

In einem Becherglas wurden mit Hilfe eines Magnetrührers die folgenden Komponenten vermischt:

| | |
|---|---|
| 348,55 g | Acrylsäure (4,84mol) |
| 135,51 g | einer 37,3 %igen Natriumacrylatlösung in Wasser (0.54mol) |
| 28 g | Polyethylenglykoldiacrylat eines Polyethylenglycols der Molmasse 400 |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azobis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 81,04 % |
| Neutralisationsgrad: | 60 mol% |
| Monomerschaumdichte: | 0,21 $gcm^{-3}$ |
| Polymerschaumdichte: | 0,20 $gcm^{-3}$ |
| Schaumstruktur: | homogen, vollständig offenzellig, keine Hautbildung |

Weitere Eigenschaften des offenzelligen Schaums sind in Tabellen 1 und 2 angegeben.

Tabelle 1

| Beispiel | FSC [g/g] | CRC [g/g] | FSR [g/gsec] |
|---|---|---|---|
| 1 | 50,3 | 11,0 | 4,9 |
| 2 | 48,2 | 10,9 | 4,6 |
| 3 | 44,4 | 11,2 | 4,2 |
| 4 | 50,0 | 10,4 | 5,5 |
| 5 | 50,5 | 8,3 | 7,9 |
| 6 | 36,0 | 10,8 | 2,9 |
| 7 | 41,7 | 12,3 | 1,9 |
| 8 | 38,5 | 19,4 | 0,2 |
| Vergleichsbeispiel 1 | 56,6 | 7,7 | 4,4 |

Tabelle 2

| Beispiel | Dicke des gequollenen Schaums [mm] | Querschnittsfläche (CSA) [$mm^2$] | Naßbruchpunkt [g] | Naßbruchwert (WFV) [$g/mm^2$] |
|---|---|---|---|---|
| 1 | 5,41 | 189,3 | 47,0 | 0,269 |

(fortgesetzt)

| Beispiel | Dicke des gequollenen Schaums [mm] | Querschnittsfläche (CSA) [$mm^2$] | Naßbruchpunkt [g] | Naßbruchwert (WFV) [$g/mm^2$] |
|---|---|---|---|---|
| 2 | 6,03 | 211,1 | 60,3 | 0,340 |
| 3 | 6,48 | 226,8 | 44,2 | 0,234 |
| 4 | 7,80 | 273,0 | 66,8 | 0,245 |
| 5 | 9,46 | 331,1 | 50,4 | 0,152 |
| 6 | 8,38 | 293,3 | 90,8 | 0,310 |
| 7 | 8,70 | 304,5 | 262,3 | 1,315 |
| 8 | 10,09 | 353,2 | 158,5 | 0,423 |
| Vergleichsbeispiel 1 | 12,60 | 441,0 | 41,0 | 0,093 |

Beispiel 9

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37.3 %igen Natriumacrylatlösung in Wasser (0,54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azobis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

In eine Form, die aus einer Glasplatte der Größe DIN A4 mit 1mm hohen Rändern bestand, legte man ein 0,69mm dickes Non-Woven Vlies aus superabsorbierenden Faser (Fiberdri P8/00 1231, ex Firma Camelot Technologies Limited, Canada) mit einer Dichte des Vlieses von 0,0995g/$cm^3$ und brachte dann darauf den zuvor hergestellten Monomerschaum auf und arbeitete ihn vorsichtig unter Erhalt der offenzelligen Schaumstruktur in das Vlies ein. Die Form wurde dann mit einer zweiten Glasplatte bedeckt. Die Schaumprobe wurde dann zwischen den beiden Glasplatten polymerisiert indem man die Glasplatten synchron von beiden Seiten mit zwei UV/VIS Strahlern (UV 1000 der Firma Höhnle) für 4 Minuten bestrahlte.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 81,35 % |
| Neutralisationsgrad: | 60 mol% |
| Monomerschaumdichte: | 0,22 $gcm^{-3}$ |
| Polymerschaumdichte mit Vlies: | 0,29 $gcm^{-3}$ |
| Schaumstruktur: | homogen, vollständig offenzellig, keine Hautbildung |
| Schaum und Vlies: | 458 $g/m^2$ |

Weitere Eigenschaften des offenzelligen Schaums sind in Tabelle 3 angegeben.

Tabelle 3

| Beispiel | Dicke des trockenen Schaums [mm] | Dicke des gequollenen Schaums [mm] | Querschnittsfläche [$mm^2$] | Naßbruchpunkt [g] | Naßbruchwert (WFV) [$g/mm^2$] |
|---|---|---|---|---|---|
| 9 | 1,75 | 3,25 | 113,75 | 25,7 | 0,226 |

Beispiel 10

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37,3 %igen Natriumacrylatlösung in Wasser (0,54mol) |
| 28 g | eines ethoxylierten Trimethylpropantriacrylats der Molmasse 956 (ETMPTA) |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 1,0 Gew.- % (4,8 g), bezogen auf Monomere, Apfelfaser (Bio-Apfelfaser AF 400, ex Firma J. Rettenmaier & Söhne GmbH & Co, Deutschland). Die erhaltene Lösung wurde in einen Druckbehälter überführt und dort für 25 min bei einem Druck von 12 bar mit Kohlendioxid gesättigt. Unter Druck wurden 26,67g einer 3%igen, wässrigen Lösung von 2,2'-Azobis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 82,13 % |
| Neutralisationsgrad: | 60 mol% |
| Monomerschaumdichte: | 0,25 $gcm^{-3}$ |
| Polymerschaumdichte: | 0,27 $gcm^{-3}$ |
| Schaumstruktur: | homogen, vollständig offenzellig, keine Hautbildung |

Weitere Eigenschaften des offenzelligen Schaums sind in Tabellen 4 und 5 angegeben.

Beispiel 11

In einem Becherglas wurden mit Hilfe eines Magnetrührers die folgenden Komponenten vermischt.

| | |
|---|---|
| 348,55 g | Acrylsäure (4,84mol) |
| 135,51 g | einer 37,3 %igen Natriumacrylatlösung in Wasser (0,54mol) |
| 28 g | Polyethylenglykoldiacrylat eines Polyethylenglycols der Molmasse 400 |
| 21,33 g | einer 15 %igen, wässrigen Lösung eines Additionsrodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90g (2,69 mol) Triethanolamin so zugegeben, daß die Innentemperatur nicht über 16°C anstieg. Zu der wäßrigen Mischung fügte man dann 1,0 Gew.-% (4,8 g), bezogen auf Monomere Apfelfaser (Bio-Apfelfaser AF 400, ex Firma J. Rettenmaier & Söhne GmbH & Co, Deutschland). Die erhaltene Lösung wurde in einen Druckbehälter überführt und dort für 25 min bei einem Druck von 12 bar mit Kohlendioxid gesättigt.

Unter Druck wurden 26,67g einer 3%igen, wässrigen Lösung von 2,2'-Azobis(2-amidinopropan)dihydrochlorid zugegeben und durch Druckerhöhung homogen untergemischt. Anschließend wurde für weitere 5 min Kohlendioxid durch die Reaktionsmischung geleitet. Die gesättigt Reaktionsmischung wurde bei einem Druck von 12 bar durch eine Düse mit einem Durchmesser von 1mm ausgepreßt, wobei sich ein feinzelliger, gut fließfähiger Schaum bildete.

Der erhaltene Monomerschaum wurde auf eine DIN A3 große Glasplatte mit 3mm hohen Rändern aufgebracht und mit einer zweiten Glassplatte bedeckt. Die Schaumprobe wurde synchron von beiden Seiten mit zwei UV/VIS - Strahlern (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf eine Feuchte von 5% eingestellt.

| | |
|---|---|
| Feststoffgehalt der Reaktionsmischung: | 82,13 % |
| Neutralisationsgrad: | 60 mol% |
| Monomerschaumdichte: | 0,22 $gcm^{-3}$ |
| Polymerschaumdichte: | 0,17 $gcm^{-3}$ |
| Schaumstruktur: | homogen, vollständig offenzellig, keine Hautbildung |

Weitere Eigenschaften des offenzelligen Schaums sind in Tabellen 4 und 5 angegeben.

Tabelle 4

| Beispiel | Teebeutel-Test (FSC) [g/g] | CRC [g/g] | FSR [g/gsec] |
|---|---|---|---|
| 10 | 43,7 | 10,1 | 1,74 |
| 11 | 55,2 | 8,1 | 6,68 |

Tabelle 5

| Beispiel | Dicke des gequollenen Schaums [mm] | Querschnittsfläche (CSA) [$mm^2$] | Naßbruchpunkt [g] | Naßbruchwert (WFV) [$g/mm^2$] |
|---|---|---|---|---|
| 10 | 6,62 | 231,7 | 89,6 | 0,387 |
| 11 | 9,31 | 325,8 | 58,8 | 0,180 |

## Patentansprüche

1. Superabsorbierende Schäume, enthaltend superabsorbierende synthetische Fasern und/oder natürliche Fasern aus der Gruppe der Apfelfasern, Orangenfasern, Tomatenfasern, Weizenfasern und/oder Haferfasern.

2. Superabsorbierende Schäume nach Anspruch 1, erhältlich durch Schäumen einer polymerisierbaren wäßrigen Mischung , die zu mindestens 50 Mol-% neutralisierte, Säuregruppen enthaltende monoethylenisch ungesättigte Monomere oder mindestens ein basisches Polymer, Vernetzer, superabsorbierende Fasern und mindestens ein Tensid enthält, und anschließendes Polymerisieren und/oder Vernetzen der geschäumten Mischung.

3. Superabsorbierende Schäume nach Anspruch 1 oder 2, **dadurch gekennzeichnet, daß** die polymerisierbare wäßrige Mischung, bezogen auf die Monomeren, 0,01 bis 10 Gew.-% an superabsorbierenden Fasern enthält.

4. Superabsorbierende Schäume nach einem der Ansprüche 1 bis 3, **dadurch gekennzeichnet, daß** die polymerisierbare wäßrige Mischung, bezogen auf die Monomeren, 0,1 bis 5 Gew.-% an superabsorbierenden Fasern enthält.

5. Superabsorbierende Schäume nach einem der Ansprüche 1 bis 4, **dadurch gekennzeichnet, daß** die Oberfläche der Schaumstoffe nachvernetzt ist.

6. Superabsorbierende Schäume nach einem der Ansprüche 1 bis 5, **dadurch gekennzeichnet, daß** die polymerisierbare wäßrige Mischung zu mindestens 50% mit Natronlauge oder Kalilauge neutralisierte Acrylsäure, einen

mindestens zwei ethylenisch ungesättigte Doppelbindungen enthaltenden Vernetzer, einen Radikale bildenden Initiator, superabsorbierende Fasern aus einem hydrolysierten und anschließend vernetzten Copolymerisat aus Isobuten und Maleinsäureanhydrid und mindestens ein Tensid enthält.

**7.** Superabsorbierende Schäume nach einem der Ansprüche 1 bis 5, **dadurch gekennzeichnet, daß** die polymerisierbare wäßrige Mischung mindestens ein basisches Polymer aus der Gruppe von Vinylamineinheiten enthaltenden Polymeren, Vinylguanidineinheiten enthaltenden Polymeren, Dialkylaminoalkyl(meth)acrylamideinheiten enthaltenden Polymeren, Polyethyleniminen, mit Ethylenimin gepfropften Polyamidoaminen und Polydiallaldimethylammoniumchloriden enthält.

**8.** Verfahren zur Herstellung von superabsorbierenden Schäumen mit verbesserter Naßfestigkeit, **dadurch gekennzeichnet, daß** man eine vernetzbare wäßrige Mischung schäumt, die zu mindestens 50 Mol-% neutralisierte, Säuregruppen enthaltende monoethylenisch ungesättigte Monomere oder mindestens ein basisches Polymer, Vernetzer, superabsorbierende synthetische Fasern und/oder natürlich Fasern aus der Gruppe der Apfelfasern, Orangenfasern, Tomatenfasern, Weizenfasern und/oder Haferfasern und mindestens ein Tensid enthält, und anschließend die in der geschäumten Mischung enthaltenen Monomeren polymerisiert oder die basischen Polymeren unter Bildung eines schaumförmigen Hydrogels vernetzt.

**9.** Verfahren nach Anspruch 8, **dadurch gekennzeichnet, daß** man eine wäßrige Mischung schäumt, die 0,01 bis 10 Gew.-%, vorzugsweise 0,1 bis 5 Gew.-% superabsorbierende Fasern enthält.

**10.** Verfahren nach Anspruch 8 oder 9, **dadurch gekennzeichnet, daß** man zum Schäumen der wäßrigen polymerisierbaren Mischung ein gegenüber Radikalen inertes Gas unter einem Druck von 2 bis 400 bar löst und die Mischung anschließend auf Atmosphärendruck entspannt.

**11.** Verwendung der superabsorbierenden Schäume nach den Ansprüchen 1 bis 7 in Hygieneartikeln zur Absorption von Körperflüssigkeiten, in Verbandmaterial zur Abdeckung von Wunden, als Dichtungsmaterial, als Verpackungsmaterial, als Bodenverbesserungsmittel, als Bodenersatzstoff, zur Entwässerung von Schlämmen, zum Eindicken wäßriger Lacke bei der Entsorgung von restlichen Lackmengen, zur Entwässerung von wasserhaltigen Ölen oder Kohlenwasserstoffen oder als Material für Filter in Lüftungssystemen.

## Claims

**1.** A superabsorbent foam comprising superabsorbent synthetic fiber and/or natural fiber selected from the group consisting of apple fiber, orange fiber, tomato fiber, wheat fiber and/or oat fiber.

**2.** The superabsorbent foam according to claim 1, obtainable by foaming a polymerizable aqueous mixture comprising at least 50 mol% neutralized acid-functional monoethylenically unsaturated monomer or at least one basic polymer, crosslinker, superabsorbent fiber and at least one surfactant and subsequently polymerizing and/or crosslinking the foamed mixture.

**3.** The superabsorbent foam according to claim 1 or 2 wherefor the polymerizable aqueous mixture contains from 0.01 to 10% by weight of superabsorbent fiber, based on the monomer.

**4.** The superabsorbent foam according to any of claims 1 to 3 wherefor the polymerizable aqueous mixture contains from 0.1 to 5% by weight of superabsorbent fiber, based on the monomer.

**5.** The superabsorbent foam according to any of claims 1 to 4, having been surface postcrosslinked.

**6.** The superabsorbent foam according to any of claims 1 to 5 wherefor the polymerizable aqueous mixture comprises at least 50% aqueous sodium or potassium hydroxide solution neutralized acrylic acid, a crosslinker comprising at least two ethylenically unsaturated double bonds, a radical-forming initiator, superabsorbent fiber composed of hydrolyzed and subsequently crosslinked copolymer of isobutene and maleic anhydride, and at least one surfactant.

**7.** The superabsorbent foam according to any of claims 1 to 5 wherefor the polymerizable aqueous mixture comprises at least one basic polymer selected from the group consisting of polymers comprising vinylamine units, polymers comprising vinylguanidine units, polymers comprising dialkylaminoalkyl(meth)acrylamide units, polyethyleneimines,

ethyleneimine-grafted polyamidoamines and polydiallyldimethylammonium chlorides.

8. A process for producing superabsorbent foam having improved wet strength, which comprises foaming a crosslinkable aqueous mixture comprising at least 50 mol% neutralized acid-functional monoethylenically unsaturated monomer or at least one basic polymer, crosslinker, superabsorbent synthetic fiber and/or natural fiber selected from the group consisting of apple fiber, orange fiber, tomato fiber, wheat fiber and/or oat fiber and at least one surfactant and subsequently polymerizing the monomer in the foamed mixture or crosslinking the basic polymer to form a hydrogel foam.

9. The process according to claim 8 wherein the aqueous mixture comprises from 0.01 to 10% by weight and preferably from 0.1 to 5% by weight of superabsorbent fiber.

10. The process according to claim 8 or 9 wherein the foaming of the aqueous polymerizable mixture is effected by dissolving a radical-inert gas at from 2 to 400 bar and subsequently decompressing the mixture to atmospheric.

11. The use of the superabsorbent foam according to any of claims 1 to 7 in hygiene articles to absorb body fluids, in dressing material to cover wounds, as a sealing material, as a packaging material, as a soil improver, as a soil substitute, to dewater sludges, to thicken waterborne paints or coatings in the course of disposing of residual quantities thereof, to dewater water-containing oils or hydrocarbons or as a material for filters in ventilation systems.

**Revendications**

1. Mousses superabsorbantes, contenant des fibres synthétiques superabsorbantes et/ou des fibres naturelles superabsorbantes du groupe des fibres de pomme, des fibres d'orange, des fibres de tomate, des fibres de blé et/ou des fibres d'avoine.

2. Mousses superabsorbantes suivant la revendication 1, que l'on peut obtenir par moussage d'un mélange aqueux polymérisable, qui contient pour au moins 50 % en moles des monomères monoéthyléniquement insaturés, contenant des groupes acides, neutralisés ou au moins un polymère basique, des agents de réticulation, des fibres superabsorbantes et au moins un agent tensioactif, et par polymérisation et/ou réticulation ultérieure du mélange mousse.

3. Mousses superabsorbantes suivant la revendication 1 ou 2, **caractérisées en ce que** le mélange aqueux polymérisable contient, par rapport aux monomères, 0,01 à 10 % en poids de fibres superabsorbantes.

4. Mousses superabsorbantes suivant l'une des revendications 1 à 3, **caractérisées en ce que** le mélange aqueux polymérisable contient, par rapport aux monomères, 0,1 à 5 % en poids de fibres superabsorbantes.

5. Mousses superabsorbantes suivant l'une des revendications 1 à 4, **caractérisées en ce que** la surface des mousses est postréticulée.

6. Mousses superabsorbantes suivant l'une des revendications 1 à 5, **caractérisées en ce que** le mélange aqueux polymérisable contient, pour au moins 50 % d'acide acrylique neutralisé par de la lessive de soude ou de potasse, un agent de réticulation contenant au moins deux doubles liaisons éthyléniquement insaturées, un amorceur formant des radicaux, des fibres superabsorbantes issues d'un copolymère hydrolysé et ensuite réticulé d'isobutène et d'anhydride d'acide maléique et au moins un agent tensioactif.

7. Mousses superabsorbantes suivant l'une des revendications 1 à 5, **caractérisées en ce que** le mélange aqueux polymérisable contient au moins un polymère basique du groupe des polymères contenant des unités vinylamine, des polymères contenant des unités vinylguanidine, des polymères contenant des unités dialkylaminoalkyl(méth) acrylamide, des polyéthylènimines, des polyamidoamines greffées avec de l'éthylènimine et des chlorures de polydiallaldiméthylammonium.

8. Procédé de préparation de mousses superabsorbantes présentant une résistance à l'état humide améliorée, **caractérisé en ce qu'**on fait mousser un mélange aqueux réticulable qui contient pour au moins 50 % en moles des monomères monoéthyléniquement insaturés, contenant des groupes acides, neutralisés ou au moins un polymère basique, des agents de réticulation, des fibres synthétiques superabsorbantes et/ou des fibres naturelles superabsorbantes du groupe des fibres de pomme, des fibres d'orange, des fibres de tomate, des fibres de blé et/ou des fibres d'avoine et au moins un agent tensioactif, et ensuite on polymérise les monomères contenus dans le mélange mousse ou on réticule le polymère basique avec formation d'un hydrogel en forme de mousse.

**9.** Procédé suivant la revendication 8, **caractérisé en ce qu'**on fait mousser un mélange aqueux qui contient 0,01 à 10 % en poids, de préférence 0,1 à 5 % en poids, de fibres superabsorbantes.

**10.** Procédé suivant la revendication 8 ou 9, **caractérisé en ce que**, pour faire mousser le mélange polymérisable aqueux, on dissout un gaz inerte vis-à-vis des radicaux sous une pression de 2 à 400 bars et on détend ensuite le mélange à la pression atmosphérique.

**11.** Utilisation des mousses superabsorbantes suivant les revendications 1 à 7 dans des articles d'hygiène pour l'absorption de liquides corporels, dans une matière de pansement pour recouvrir des plaies, comme matière d'étanchéification, comme matière d'emballage, comme produit d'amélioration des sols, comme substitut de sol, pour la déshydratation de boues, pour l'épaississement de vernis aqueux lors de l'élimination de quantités de vernis résiduaires, pour la déshydratation d'huiles ou d'hydrocarbures contenant de l'eau ou comme matière de filtre dans des systèmes d'aération.

FIG. 1

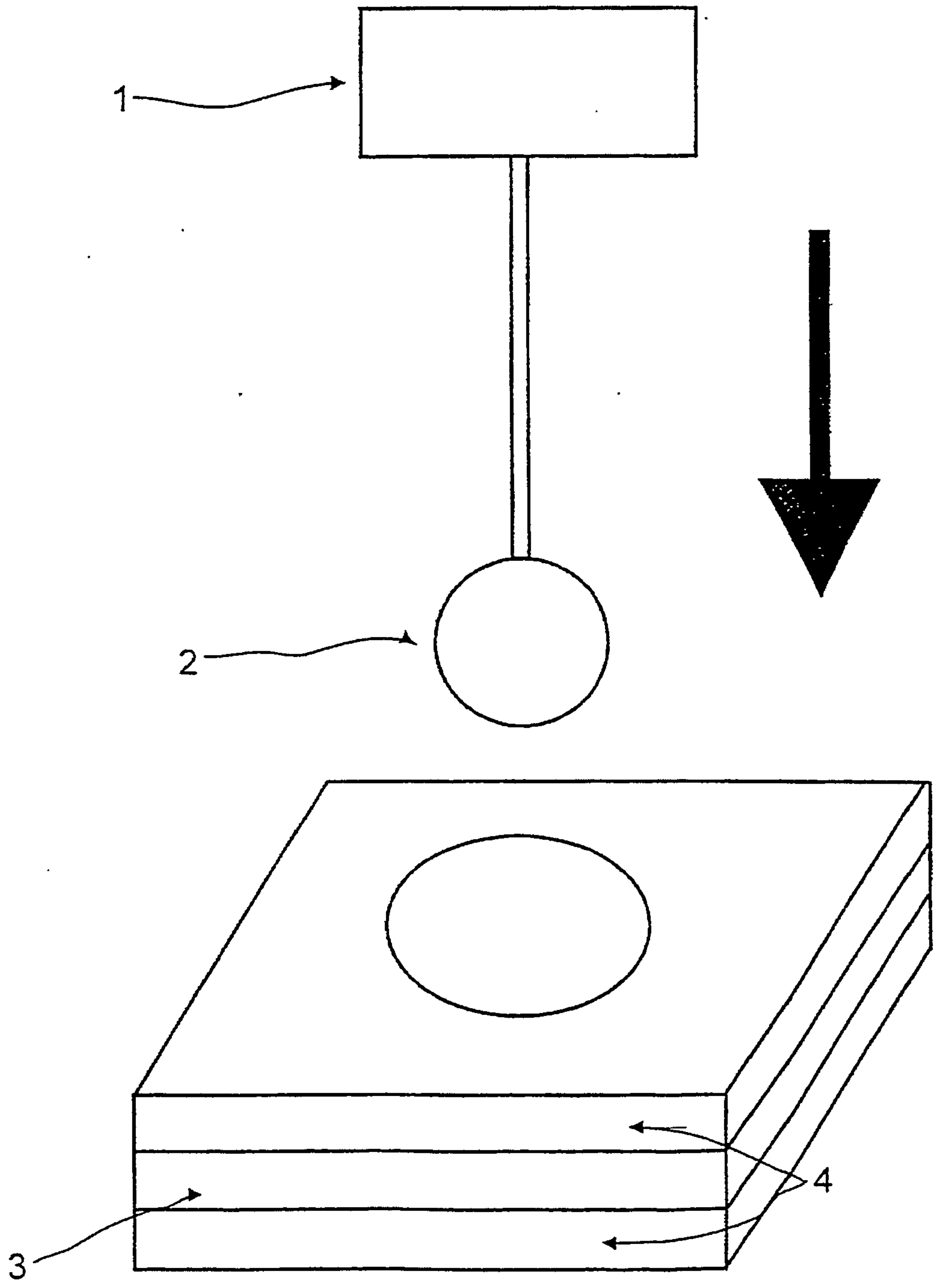

FIG.2

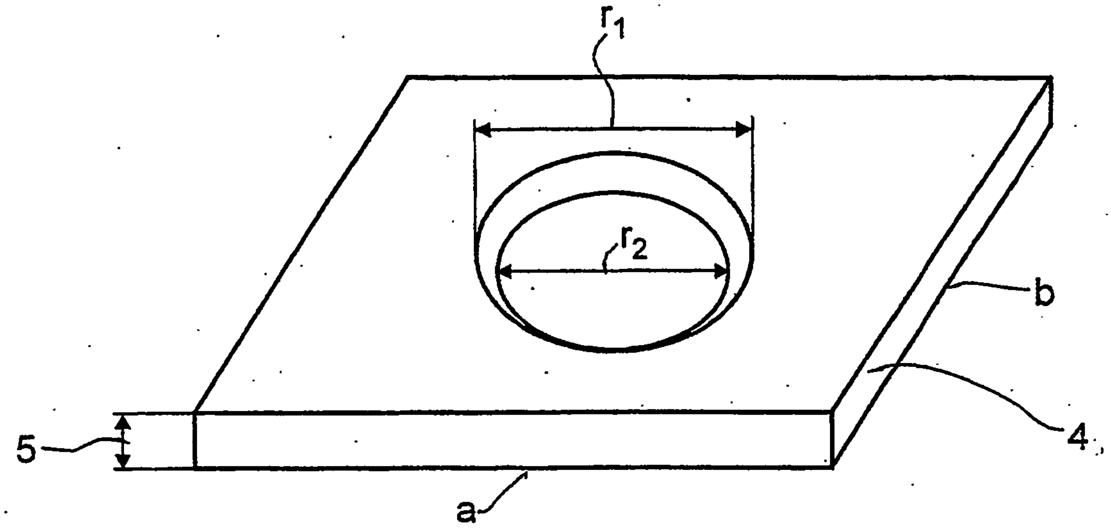


FIG.3